(19)

(11) **EP 4 467 551 B1**

(12) **EUROPEAN PATENT SPECIFICATION**

(45) Date of publication and mention of the grant of the patent:
**25.02.2026 Bulletin 2026/09**

(21) Application number: **23174575.3**

(22) Date of filing: **22.05.2023**

(51) International Patent Classification (IPC):
***C07F 5/05*** (2006.01) ***C07F 5/00*** (2006.01) ***C07D 201/00*** (2006.01) ***H10K 85/30*** (2023.01) ***C09K 11/06*** (2006.01) ***C07D 498/08*** (2006.01) ***C07D 498/18*** (2006.01) ***C07D 498/22*** (2006.01)

(52) Cooperative Patent Classification (CPC):
**C07F 5/003; C07D 498/08; C07D 498/18; C07D 498/22; C07F 5/05; C09K 11/06; H10K 85/351;** H10K 50/11

(54) **METAL-ORGANIC COORDINATION COMPOUND AND METHOD FOR PRODUCING THE SAME**

METALLORGANISCHE KOORDINATIONSVERBINDUNG UND VERFAHREN ZU DEREN HERSTELLUNG

COMPOSÉ DE COORDINATION ORGANOMÉTALLIQUE ET SON PROCÉDÉ DE PRODUCTION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**27.11.2024 Bulletin 2024/48**

(73) Proprietor: **beeOLED GmbH**
**01217 Dresden (DE)**

(72) Inventors:
- **Mewes, Jan-Michael**
**53129 Bonn (DE)**
- **Barthel, André**
**01159 Dresden (DE)**
- **Kaden, Felix**
**01127 Dresden (DE)**

(74) Representative: **Altmann, Andreas**
**Altmann Stößel Dick Patentanwälte PartG mbB**
**Isartorplatz 1**
**80331 München (DE)**

(56) References cited:
**EP-A1- 4 161 941** **WO-A1-2022/218562**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 467 551 B1

## Description

### Technical Field

The invention concerns a metal-organic coordination compound, wherein the coordination compound comprises one lanthanide ion selected from Eu(II) coordinated by a polycyclic organic ligand, a mixture containing the metal-organic coordination compound, an organic electronic device containing the coordination compound or the mixture, and methods for forming the device in particular using thermal evaporation or sublimation or casting from solution.

### Background

Electroluminescent devices that make use of organic light emitting diodes (OLEDs) are state-of-the-art for flat panel display applications used in everyday consumer electronics. For OLEDs, special organic materials, so-called emitters, are employed for the purpose of converting electrical excitation into light emission. For most organic emitters, the excitation that is formed upon recombination of an electron and a hole on is called an "exciton". Depending on the spins of the recombining charges, there are two types of excitons formed in a statistical manner: 75% probability of triplet excitons with spin 1, and 25% probability singlet excitons with spin zero are generated. If the emitter molecule is heteroaryl-based without any significant content of heavy metals, than, because of quantum mechanical selection rules that ensure spin-conservation, only the singlet excitons contribute to light emission via fluorescence (FL), whereas all triplets decay non-radiatively. Thus, fluorescent OLEDs are comparably inefficient as 75% of the invested electrical power is wasted.

In a related art, the quantum mechanical heavy-atom effect is used by introducing fourth and fifth row d-block metal elements, such as Iridium, Osmium, Gold or Platinum into the emitter. The presence of such heavy elements softens the selection rules for the excited states in the emitters, allowing also triplet excitons to emit light on useful timescales; known as phosphorescence (Ph).

In another related art, thermally-activated delayed fluorescence (TADF) has been used, wherein by thoughtful design of the organic emitter molecule, the energy difference between the non-emissive triplet and the emissive singlet exciton is engineered to be very small. This allows triplets to thermally up-convert into singlet excitons via the so-called reverse intersystem-crossing process, and thereby contribute to light emission.

However, there are no TADF or Ph blue emitters with sufficient chemical stability known which hinders their implementation into OLED consumer applications. The underlying cause of low chemical stability is the formation of charge-separated states upon excitation. For Ph emitters, the exciton resides on two different parts of the emitter, namely the central heavy metal cation and the organic ligand. During excitation, especially with high energetic blue light, the chemical bond between metal center and organic ligand is weakened, giving rise to chemical decomposition. Similarly, for TADF emitters, a low energy difference between singlet and triplet exciton energy is needed, which is achieved by bridging the excitation in-between an electron accepting and an electron donating part. Again, upon excitation with energies corresponding to blue photons, the organic bond between those two parts of the emitter molecule is substantially weakened, giving rise to bond cleavage and, in consequence, short operational lifetime in OLED device. Therefore, TADF or Ph blue emitting materials cannot be used in display applications, as otherwise the blue spectral component would fade away after prolonged operation times, which is known as burn-in.

Thus, for emitter molecules suitable for OLED, it is desirable to avoid charge-separated states, and instead localize the excitation on one part of the molecule, preferably on a single atom. Here intrametallic transitions of lanthanides offer an elegant solution as the excitation is confined within a single atom and, as such, cannot weaken any organic bond. The visible optical transitions of most lanthanides occur within the same orbital; they are of f-f type. As such, f-f transitions do not involve a change of the symmetry of the involved orbitals, which makes them quantum mechanically (parity) forbidden, resulting in a comparably long excited state lifetime of around one millisecond. This renders them unsuitable for consumer display applications, which require fast content refresh rates and high brightness - in other words: high excitation densities. In addition to f-f transitions, some lanthanides feature parity-allowed d-f type transitions, which, in turn, possess sufficiently short excited state lifetime of around one microsecond or less. These suitable lanthanides are Eu(II), Ce(III), Sm(II), and Yb(II). Divalent Samarium is red emitting and, as such, not interesting for display applications. The ground state of divalent Ytterbium is a close shell configuration, with the excited state being a blue emitting singlet. Yet a lower triplet excitation may form by inter system crossing, which is spin-forbidden showing reduced luminescence quantum yield and is not of blue color anymore. Trivalent Cerium features a single electron configuration resulting in a doublet of two distinct spectral peaks, separated by typically 80 nm. However, for display applications, pure emission colors are required or preferable. Therefore, large parts of the emission must be filtered away, which effectively reduces the maximum achievable power efficiency. Finally, for divalent Europium, the photophysical situation is very favorable: The ground state configuration features a half-filled 4f-orbital, resulting in a single Gaussian peak. This is at the same time the lowest optical transition; no undesired longer-lived states are possible. Chemical engineering of the organic ligand allows to tune the emission color through the whole visible spectrum; especially using electronically hard ligands deep blue and color pure

emission, i.e with a narrow spectral band width, and high photoluminescence quantum yield, that are ideally suited for display applications, have readily been disclosed in prior art WO 2021/244801 A1.

As mentioned above, for intrametallic transitions, the excitation energy is confined on a single atom and does not weaken any chemical bonds. Yet, during electroluminescent device operation, charge-trapping on Eu frequently occurs, which corresponds to an oxidation of Eu(II) to Eu(III). Thus, another mandatory requirement for stable OLED operation is a reversible oxidation/reduction, requiring a stabilization of Eu(II) over Eu(III). This creates a challenge, since the most stable oxidation state of Eu is typically the trivalent one. In other words, a stabilization of oxidized trivalent europium after it has captured a positive charge must be avoided. This is being accomplished by using rigid coordination environments and hard anions. In this respect, macrocyclic and in particular polymacrocyclic ligands (cryptands) are well suited to provide a rigid chemical environment. Since the ionic radius of Eu(III) is significantly smaller than that of Eu(II), adapting the cavity size of the macrocyclic ligand specifically to Eu(II) stabilizes the divalent over the trivalent oxidation state. By coordinating divalent europium using rigid polymacrocyclic ligands, in particular nitrogen-rich cryptands, yellow-green (Chem. Commun., 2018, 54, pages 4545-4548) and blue (WO 2021/244801 A1) electroluminescent devices have been demonstrated.

Although OLEDs have been demonstrated with such emitters, those particular emitters cannot be employed for the mass production of consumer displays. Today the vast majority of all consumer OLED displays is produced using thermal evaporation or sublimation, whereas the green-emitting devices disclosed in Chem. Commun., 2018, 54, pages 4545-4548 were processed using thermal sublimation. However, the sublimation yield of those particular emitters was only 10%. Additionally, significant thermal decomposition has been observed during the sublimation process, which renders this material not suitable for mass production. WO 2022/218562 discloses, twofold negatively charged polymacrocyclic ligands for Eu(II) for improved thermal vacuum sublimation. However, again only green emitting Eu(II) emitter are practiced and, once more, the sublimation yield is well below 100%. Thus, today no blue emitting Eu(II) coordination compound using a size discriminating macrocyclic ligand is known that can be processed by thermal evaporation or sublimation with high yield and without significant decomposition.

Alternatively to gas transfer, OLED emitting layers may as well be processed using wet solution coating, for example via ink-jet printing. Using this process, the deep-blue-emitting devices disclosed in WO 2021/244801 A1 have been exemplified. Clearly, the performance of this device is not yet convincing, showing relatively low luminance. The issue here is the use of highly polar solvents to cast the emission layer, which partly redissolves the underlying charge transport layers causing high leakage currents and charge trapping in the seed layers. In fact, high quality solution processed OLEDs require the use of non-polar solvents such as toluene or tetrahydrofuran for the processing of the emission layer to keep the hole transporting seed layers, for example PEDOT-PSS, intact. As of today, no blue-emitting Eu(II) coordination compound using a size discriminating macrocyclic ligand is known that can be solution processed using low polar solvent such as toluene or tetrahydrofuran.

It is an objective of the present invention to provide a deep-blue-emitting metal organic coordination compound suitable for mass produced consumer electroluminescence applications using solution or gas transfer coating. In particular, the objective is to provide a polymacrocyclic coordinated lanthanide, preferably Eu(II) emitter that is blue emitting and that can be processed by thermal evaporation or sublimation with high yield and without significant decomposition. Furthermore, it is another particular objective of this invention to provide a polymacrocyclic coordinated lanthanide, preferably Eu(II) emitter that is blue emitting and that can be processed from solution using a low polar solvent, such as toluene or tetrahydrofuran.

The objective is achieved according to the present invention by a metal-organic coordination compound, wherein the coordination compound comprises one lanthanide ion selected from Eu(II) coordinated by a polycyclic organic ligand having the formula (1-1) or (1-2)

(1-1) (1-2)

with

- $\mathbf{L_0, L_1, L_2}$ being independently each a divalent organic group formed by removing two hydrogen atoms from an organic molecule containing at least two hydrogen atoms, with
- the shortest sequence of atoms in L1, and L2 each linking the two nitrogen atoms adjacent to L1, and L2 being each 5 atoms long, which are carbon atoms, with one non-terminal carbon atom of these being replaced by O, and with

- **R** independently in each occurrence representing hydrogen, deuterium, halogen, or a monovalent organic group formed by removing a hydrogen atom from an organic molecule containing at least one hydrogen atom, and herein any two beta- or gamma-positioned R can be linked to each other to form a ring structure, and by

a mixture comprising

(a) the coordination compound according to the invention, and
(b) at least one second electrically neutral organic compound different therefrom,

wherein the coordination compound (a) is imbedded into the at least one second electrically neutral organic compound (b), which preferably is a charge-neutral host material,
wherein the second organic compound (b) has a triplet energy higher than 2.5 eV, preferably higher than 2.6 eV and more preferably higher than 2.7 eV, wherein compounds (a) and (b) are physically mixed or wherein the second charge-neutral compound (b) has a molecular weight $M_n$ above 1000 g/mol and the coordination compound (a) and the second charge-neutral compound (b) are chemically linked with each other, for example by a covalent or coordinative chemical bond, and by
an organic electronic device, comprising:

a first electrode;
a second electrode; and
an organic layer arranged such that it is electrically interposed between the first and second electrodes, wherein the organic layer comprises the coordination compound, or the mixtures according to the invention,
wherein the organic electronic device is preferably an organic light emitting diode,
whereby preferably a substantial portion of the light is emitted at wavelengths lower than 500 nm, and by
a method of forming an organic device according to the invention, the method comprising:

forming a layer of the coordination compound, or of the mixtures according to the invention,
wherein the layer is deposited from a gas phase, preferably using an evaporation and/or sublimation and/or carrier gas process, or wherein the layer is deposited from solution, and by
the use of the coordination compound or a mixture according to the invention as active emitting material in electroluminescent electronics applications or as a dye or contrast enhancement medium for magnetic resonance tomography.

In particular, it was found that in the prior art coordination compounds the cavity size of the mostly symmetrical polycyclic organic ligands did, in fact, not match the central cation well, as it was often far too large. Mostly, too large cavity diameters have been employed, since the polycyclic organic ligands were typically based on ring sizes containing 18 atoms. Thus, it is part of this invention to provide geometric stabilization using a rigid polycyclic organic ligand with a cavity size that perfectly matches the size of the central cation. This is achieved by allowing only 4-6 atoms as the shortest sequence of $\mathbf{L_1}$ and $\mathbf{L_2}$ between the two nitrogen bridge head atoms shown in formula (1-1) and (1-2). Additionally, the ligand contains a plurality of hard donor atoms to achieve electronic stabilization and deep blue color point. Furthermore, a feature of the present invention is to provide distinct asymmetrical polycyclic organic ligands, since one of the chemical linkages between the two nitrogen bridge-head atoms is significantly longer than the other two.

Indeed, straightforward syntheses of various specific examples of the type of compounds according to formula (1-1) have been described in literature (compare for examples: R. W. Taylor et al. J. Chem. Crystallogr. 2012, 42, 573-577; B. Valtoncoli et al. in J. Chem. Soc. Perkin Trans. 2, 1994, 815-820 and J. Chem. Soc. Perkin Trans. 2, 1994, 3581-3588; J. Jurczak et al. SYNTHESIS 2004, 3, 0369-0372; R. W. Taylor et al. Acta Cryst. 2008, 64, 2003). However, these prior art references did not recognize or mention that the core structure according to (1-1) is particularly well-suited for lanthanide complexation, especially divalent lanthanides, due to its size, low polarizability, high triplet energy, and asymmetrical nature with two large and one small rings.

The desired asymmetry allows two negatively charged anionic species to integrate into the coordination compound (complex) in a way that they simultaneously stabilize the central lanthanide and partially cancel the dipole moment of the overall complex, especially in the case of twofold positively charged central cations such as divalent europium, which is preferably employed. In this way, a chemically robust blue-light-emitting complex is obtained, which, at the same time, can be processed using low polar solvents. Alternatively, the low dipole moment improves the volatility of the compounds, giving rise to high transfer yields using thermal evaporation or thermal sublimation methods. The electroluminescent coordination compounds according to formulae (1-1) or (1-2) are thus ideally suited as deep blue emitters for high efficiency OLED display applications that can be processed using low-cost sublimation, evaporation or

solution coating techniques.

## Summary of the invention

In various aspects of this invention metal-organic coordination compounds, mixtures containing the metal-organic coordination compounds, organic electronic devices comprising the metal-organic coordination compounds as well as methods for forming the same are provided.

The object is achieved by a metal-organic coordination compound as defined above and in claim 1.

According to structure (1-1) and (1-2), $\mathbf{L_1}$ and $\mathbf{L_2}$ are connected by two nitrogen atoms which are adjacent to $\mathbf{L_1}$ and $\mathbf{L_2}$ and which together form a basic cyclic group, herein referred to as basis ring. The two nitrogen atoms are further substituted with two oxygen-containing groups with the oxygens being covalently linked to each other, finally forming a polycyclic organic ligand with two characteristic nitrogen and oxygen atoms each. In formula (1-1), the two oxygen atoms are part of a possibly substituted aliphatic hetero-alkyl chain, whereas in formula (1-2) the oxygens are part of an aromatic furanyl group each. The length of $\mathbf{L_0}$, $\mathbf{L_1}$ and $\mathbf{L_2}$ can be fine-tuned to optimally fit the size of a lanthanide cation, thereby providing high stabilization. However, to achieve stable complexes, the basis ring must be smaller than a 18-crown-6 motive and as such the shortest sequences of $\mathbf{L_1}$, $\mathbf{L_2}$ must be 5 atoms. Additionally, for good complexation $\mathbf{L_1}$, $\mathbf{L_2}$ must contain each at least one complexing non terminal heteroatom selected from O. Hard donor-atoms cause less undesirable polarization effects in excited state, so preference is given to the heteroatom O. R independently in each occurrence represents halogen, hydrogen, or deuterium atoms, or a monovalent organic group formed by removing a hydrogen atom from an organic molecule containing at least one hydrogen atom, and herein any two beta- or gamma-positioned R can be linked to each other to form a ring structure. Polarization in the excited state often lowers the emission quantum efficiency. So low polarization using hard heteroatoms as part of the complexing ligand is preferred. This is preferably achieved by designing the complexing parts $\mathbf{L_0}$, $\mathbf{L_1}$ and $\mathbf{L_2}$ of a polycyclic ligand according to formula (1-1) or (1-2) preferably only contain atoms selected from hydrogen, and/or deuterium, carbon, oxygen and nitrogen.

To avoid geometric relaxation, leading to undesired Stokes Shifts and large stabilization of Eu(III) over Eu(II), rigid ligands are generally preferred. This may be achieved by incorporation of aryl or heteroaryl groups as part of the complexing parts of the polycyclic ligand, i.e. in $\mathbf{L_0}$, $\mathbf{L_1}$ and $\mathbf{L_2}$. Such aromatic units may as well improve the charge transport or charge capture properties of the metal organic coordination compounds. Yet, preference is given to aromatic groups with sufficiently high triplet energy to have the excitation being confined on the central metal and, again, preference is given to relatively strong electron-donating aromatic groups comprising none, or only oxygen or nitrogen as heteroatoms. Therefore, aromatic building blocks as part of $\mathbf{L_0}$, $\mathbf{L_1}$, and $\mathbf{L_2}$ are preferably selected from benzene, pyridine, pyrrole, pyrazole, furan, carbazole, dibenzofuran, benzimidazole, or benzofuran, preferably furan or pyrrole, whereby preference is given to furan as this particular one is the hardest heteroaryl group that still has favorable complexing character.

The (hetero) aromatic units can be substituents to the shortest sequence of atoms in $\mathbf{L_0}$, $\mathbf{L_1}$ and $\mathbf{L_2}$, or can preferably form part of this sequence. Therefore, they are derived from the mentioned (hetero) aromatic compounds by removing one or two hydrogen atoms.

In a further preferred embodiment the shortest sequences of $\mathbf{L_1}$, and $\mathbf{L_2}$ in (1-1) and (1-2) are 5 atoms long are selected from ethyleneoxy, azaethylene or dimethylfuran fragments and the polycyclic organic ligand is selected from one of formulas (1-3), (1-5), (1-6), or (1-8)

1-3

1-5

1-6

1-8

This molecular design ensures a favorable size of the basis ring, i.e., the cyclic structure comprising the two shortest sequences of atoms of $\mathbf{L_1}$ and $\mathbf{L_2}$ linked by the two bridging nitrogen atoms has 12 atoms. Simultaneously, those symmetrical basis rings are preferably exclusively comprised of low polarizable complexing units, i.e. nitrogen, oxygen, and furan. As said above, preference is generally given to electronically hard ligands. Preferred examples that only use oxygen as complexing atoms in the form of ethyleneoxy, or dimethylfuran are given in formula (1-9) - (1-21) for the polycyclic organic ligands:

1-9 1-10 1-11 1-12 1-13

1-14 1-15 1-16

1-17 1-18 1-19

1-20 1-21

with $R_b$ independently in each occurrence representing hydrogen, deuterium, halogen - preferably chlorine - or straight or branched alkyl chain radicals including cycloalkyl radicals, the alkyl groups containing from one to fifteen, preferably one to ten, more preferably one to five carbon atoms, the term "cycloalkyl radical" referring to monocyclic, polycyclic, and spiro alkyl radicals containing 3 to 12, preferably 3 to 8 ring carbon atoms , and **n** independently in each occurrence being an integer in the range of 0 to 10, preferably 1 to 3. In those preferred examples not just the basis ring only comprises of oxygen as heteroatom but the third part of the ligand, $L_0$, as well. Here, $L_0$ may comprise a simple ethyleneoxy sequence, or a furan, or a dibenzofuran group. In case of the ethyleneoxy sequence, a better rigidity may be achieved by "fused" benzene rings which replace the 1,2-ethylene group with a 1,2-benzene(yl) group as exemplified in (1-12) and (1-17). In general, and preferred, the aromatic groups, such as benzene or furan, are arranged such that the triplet energy of the ligand is higher than the lowest excitation energy of the central lanthanide metal to ensure that the exciton is confined on the central metal. Similarly, preference is given to ligand designs with low electron and hole affinity, such that exciton is confined on the central metal ion, or, in other words, that the excited state does not attain any charge transfer character. For some applications, it may be preferred to complex the central metal cation with a singly or multiply negatively charged ligand in which the negatively charged groups are covalently linked with the ligand. Here, the ligand may comprise covalently linked singly negatively charged anionic groups. While there are many possibilities for such anionic groups, preference is generally given to substituted or non-substituted closo-borane- or closo-carborane clusters. Preferred examples of twofold negatively charged ligands comprising covalently attached carborane cluster as anions are given in formula (1-16), (1-19), and (1-21). They give neutrally charged complexes with Eu(II).

Eu(II) can emit in the deep blue spectral region, which is highly beneficial for electroluminescent applications. At the same time, the transition of Eu(II) cations is of parity-allowed d-f type, which ensures sufficiently short lifetimes of the excited state., Eu(II) combines a color-pure deep blue and single peak emission spectrum, which is optically allowed and as such Eu(II) has blue d-f optical transitions.

In various embodiments the coordination compound comprises a polycyclic organic ligand according to formula (1-1), or (1-2) and at least one negatively charged anion that is not covalently linked to the polycyclic organic ligand. This is to ensure charge neutrality once the organic ligand is coordinating the positively charged cation or cations. Preferably, the coordination compound is neutrally charged. If, for example, the polycyclic organic ligand is neutrally charged and the lanthanide is Eu(II), then two singly negatively charged anions or one doubly negatively charged anion is necessary to form the overall charge-neutral coordination compound. This negatively charged counterion or anion can be any desired anion that fulfils the above-mentioned requirements, i.e., having sufficiently high triplet energy and low polarizability. If the polycyclic organic ligand is singly (or doubly) negatively charged by virtue of a substituent (or two), only one (or no) additional negatively charged anion is necessary to neutralize the Eu(II) coordination compound.

Whilst any anions can be chosen for charge neutrality, of particular interest are those with sufficiently high triplet energy >2.7 eV so that the excitation is confined to the metal-organic complex. Additionally, preferred anions do not (significantly) absorb in the visible spectral region and, as such, do not reabsorb any light generated by the central metal cation. Additionally, preferred anions do not (significantly) polarize the excited metal centered state, i.e., they are chemically soft. Such anions with the desired properties are preferably selected from halogen, preferably iodide or *closo*-carborane clusters, e.g. according to formula (1-22) and (1-23) or nido-borane clusters, e.g. according to formula (1-24):

(1-22)
(1-23)
(1-24)


with $\mathbf{R_b}$ independently in each occurrence representing hydrogen, deuterium, halogen - preferably chlorine, straight or branched chain or cyclo-$C_{1-15}$-alkyl-preferably methyl.

In another embodiment, the polycyclic ligand according to formula (1-1) or (1-2), excluding the lanthanide, is itself at least singly, preferably doubly, negatively charged. In preferred embodiments, no free anions are present in the coordination compound according to the invention. In other words, in a combination of the ligand, (e.g. according to formula (1-1) or (1-2)) with at least one lanthanide cation, the ligand compensates the charge of the lanthanide, forming overall a complex of neutral charge. Thus, considering a divalent Europium as central metal, the preferred ligand would be twofold negatively charged. Such compounds without free (i.e. non-covalently bonded) negatively charged anions might be beneficial in electroluminescent device applications, where those very free anions may drift during operation in high electrical fields, leading to short device lifetimes.

The negative charge of a ligand may be introduced by anionic side groups (refer to formula (1-16), (1-19) or (1-21). Again, in view of (sufficiently) high triplet level, low polarizability, and (sufficiently) high energy levels, preference is given to ligands comprising at least one, or - in view of complexing a divalent Europium - preferably two covalently attached closo-carborane fragments according to formula (1-25) or (1-26):

(1-25)
(1-26)


wherein the dashed line indicates the covalent linkage to the remainder of the organic ligand, and with $R_b$ independently in each occurrence represents either hydrogen, deuterium, halogen - preferably chlorine , - -, or straight or branched alkyl chain radicals including cycloalkyl radicals, the alkyl groups containing from one to fifteen, preferably one to ten, more preferably one to five carbon atoms, the term "cycloalkyl radical" referring to monocyclic, polycyclic, and spiro alkyl radicals containing 3 to 12, preferably 3 to 8 ring carbon atoms preferably $C_{1-3}$- alkyl, more preferably methyl. A particular advantage of those types of anionic side groups is their high chemical stability, ensuring stability of the overall metal organic complex.

In another set of embodiments, the coordination compound and a second electrically neutral organic compound form a mixture, wherein the coordination compound (a) is imbedded into the at least one second electrically neutral organic compound (b), wherein the second organic compound (b) has a triplet energy higher than 2.5 eV, preferably higher than 2.6 eV and even more preferably higher than 2.7 eV, wherein compounds (a) and (b) are physically mixed or wherein the second charge-neutral compound (b) has a molecular weight Mn above 1000 g/mol and the coordination compound (a) and the second charge-neutral compound (b) are chemically linked with each other, for example by a covalent or coordinative chemical bond.

In applications of metal organic complexes, the material is very often used in combination with other organic materials, for example to ensure charge transport or proper isolation of the metal organic complex according to the

invention. This embodiment ensures that the excited state of the emitter is confined on itself. Here, if the triplet energy level of the surrounding material is too low, excitation-energy transfer will cause low light generation efficiencies.

In another set of embodiments, the coordination compound (a) and a second electrically neutral organic compound (b) form a mixture, wherein the coordination compound is imbedded into the at least one second electrically neutral organic compound in a ratio of 0.1 to 99 vol%, based on the mixture which is 100 vol%, wherein the second organic compound has a lower hole affinity compared to the neutral coordination compound, and/or wherein the second organic compound has a lower electron affinity compared to the singly positively charged coordination compound. This embodiment ensures that the excited state of the emitting molecule is electronically confined on the emitter. Essentially, the second matrix material is neither accepting electrons nor holes from the excited emitter molecules, thereby avoiding the formation of undesired charge-transfer states that compromise color-purity and light-emission efficiency.

In another embodiment, the coordination compound is used in an organic electronic device, comprising a first electrode; a second electrode; and an organic layer arranged such that it is electrically interposed between the first and second electrodes, wherein the organic layer comprises the coordination compound, or the mixture with the coordination compound. In a preferred embodiment, this organic electronic device is a light emitting diode intended to convert electrical energy into visible light. In an even preferred embodiment, the light emission of the organic light emitting diode occurs substantially at wavelength shorter than 500 nm. In other words, the electroluminescent device emits mostly, in other words has its spectral peak luminance, in the desired deep blue spectral region needed for consumer display applications.

Preferably, the coordination compound applied in the organic electronic device is deposited from the gas phase, in particular using an evaporation and/or sublimation and/or carrier gas process. Thermal sublimation and/or evaporation are the standard processes used in industrial consumer display production processes.

Alternatively, the coordination compound applied in the organic electronic device is deposited from solution, which may offer advantages in terms of throughput or production cost. In preferred embodiments, such solutions comprise substantially organic solvents with a polarity index of less than 5.0, preferably of less than 4.0, and even more preferably of less than 3.0. The polarity index of all common organic solvents can be readily found in scientific literature, for example https://people.chem.umass.edu/xray/solvent.html. This ensures that the always present underlying organic layers remain intact and functional after processing the organic layer comprising the metal organic compound according to the invention.

## Brief Description of the Drawings

In the drawings, like reference characters generally refer to the same parts throughout the different views. The drawings are not necessarily to scale, emphasis is instead generally being placed upon illustrating the principles of the invention. In the following description, various aspects of the invention are described with reference to the following drawings, in which:

**FIG. 1** exemplifies ligands according to the invention, with reference ligands L16, L26, L27, L33-44, L48-56, L74-88, L95 not being according to the present invention.

**FIG. 2** illustrate formulas of various possible side groups or substituents or parts of organyl groups of polycyclic organic ligands of the present invention.

**FIG. 3** illustrate formulas of various covalently bound anionic groups which can be present in the metal-organic complex according to the invention. The dotted line is the bond to the polycyclic organic ligand.

**FIG. 4** illustrate formulas of various non-covalently bound anionic groups which can be present in the metal-organic complex according to the invention.

**FIG. 5** exemplifies metal-organic complexes according to the invention with reference complexes E11, E17, E44-47 not being according to the present invention.

**FIG. 6** and **FIG. 7** illustrate schematic cross sections of organic electronic devices according to various aspects.

**FIG. 8** and **FIG. 9** depict emission spectra of various complexes according to the invention.

**FIG. 10** show OLED device characteristics using a complex according to the invention as active emitter.

## Detailed Description

The following detailed description refers to the accompanying drawings that show, by way of illustration, specific details and aspects in which the invention may be practiced.

The word "exemplary" is used herein to mean "serving as an example, instance, or illustration". Any aspect or design described herein as "exemplary" is not necessarily to be construed as preferred or advantageous over other aspects or designs.

Various embodiments relate to metal organic compounds, including a lanthanide cation coordinated by a polycyclic organic ligand including at least two aliphatic amine groups and two (ring) oxygens and the applications of those compounds.

In this description, a coordination compound is taken to mean a compound where the central active metal is coordinated without a direct metal carbon bond. The terms complex and coordination compound will be used interchangeably.

In this description, the arylene is a divalent organic fragment that is derived from an aromatic or heteroaromatic hydrocarbon (arene or heteroarene) by removing two hydrogen atoms from the aromatic or heteroaromatic hydrocarbon, preferably from different carbon and/or hetero atoms. One example is a (hetero) aromatic hydrocarbon that has had hydrogen atoms removed from two, preferably adjacent, hydrogen-bearing atoms (in case of aromatic hydrocarbon two carbon atoms, in case of heteroaromatic hydrocarbons two atoms selected from carbon and heteroatoms). An aromatic hydrocarbon or arene (or sometimes aryl hydrocarbon) is a cyclic hydrocarbon comprising only $sp^2$-hybridized carbon atoms, leading to a delocalized pi-system. An alkylene is a divalent organic fragment that is derived from an alkyl group by removing one additional substituent.

The terms "halo," "halogen," and "halide" are used interchangeably and refer to fluorine, chlorine, bromine, and iodine.

The term "acyl" refers to a substituted carbonyl radical (C(O)-Rs).

The term "ester" refers to a substituted oxycarbonyl (-O-C(O)-Rs or - C(O)-O-Rs) radical.

The term "ether" refers to an -ORs radical.

The terms "sulfanyl" or "thio-ether" are used interchangeably and refer to a - SRs radical.

The term "sulfinyl" refers to a -S(O)-Rs radical.

The term "sulfonyl" refers to a -SO2 -Rs radical.

The term "phosphino" refers to a -P(Rs)3 radical, wherein each Rs can be same or different.

The term "silyl" refers to a -Si(Rs)3 radical, wherein each Rs can be same or different.

In each of the above and in all occurrences of R in the formula, Rs can be hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, and combination thereof. Preferred Rs are selected from the group consisting of alkyl, cycloalkyl, aryl, heteroaryl, and combination thereof.

The term "alkyl" refers to and includes both straight and branched alkyl chain radicals and also includes cycloalkyl radicals. The alkyl groups contain from one to fifteen, preferably one to ten, more preferably one to five carbon atoms and includes methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl, and the like. Additionally, the alkyl group is optionally substituted, e.g. by halogen or cycloalkyls.

The term "cycloalkyl radical" refers to and includes monocyclic, polycyclic, and spiro alkyl radicals. Preferred cycloalkyl groups are those containing 3 to 12, preferably 3 to 8 ring carbon atoms and includes cyclopropyl, cyclopentyl, cyclohexyl, bicyclo[3.1.1]heptyl, spiro[4.5]decyl, spiro[5.5]undecyl, adamantyl, and the like. Additionally, the cycloalkyl group is optionally substituted, e.g. by halogen, deuterium, alkyl or heteroalkyl.

The terms "heteroalkyl" or "heterocycloalkyl" refer to an alkyl or a cycloalkyl radical, respectively, having at least one carbon atom replaced by a heteroatom. Preferably the at least one heteroatom is selected from O, S, N, P, B, Si and Se, more preferably O, or N. Preferably 1 to 5, more preferably 1 to 3, most preferably 1 or 2 heteroatoms are present in the radical. The radical can be covalently linked with the remainder of the molecule via a carbon or heteroatom (e.g. N). Additionally, the heteroalkyl or heterocycloalkyl group is optionally substituted as indicated for alkyl and cycloalkyl.

The term "alkenyl" refers to and includes both straight and branched chain alkene radicals. Alkenyl groups are essentially alkyl groups with more than one carbon atom that include at least one carbon-carbon double bond in the alkyl chain. Cycloalkenyl groups are essentially cycloalkyl groups that include at least one carbon-carbon double bond in the cycloalkyl ring. The term "heteroalkenyl" as used herein refers to an alkenyl radical having at least one, preferably 1 to 5, more preferably 1 to 3, most preferably 1 or 2 carbon atoms replaced by a heteroatom. Preferably, the at least one heteroatom is selected from O, S, N, P, B, Si, and Se, more preferably O, S, or N. Preferred alkenyl/cycloalkenyl/heteroalkenyl groups are those containing two/three/one to fifteen carbon atoms. Additionally, the alkenyl, cycloalkenyl, or heteroalkenyl group is optionally substituted, as indicated above.

The terms "aralkyl" or "arylalkyl" are used interchangeably and refer to an alkyl group that is substituted with an aryl group. Additionally, the aralkyl group is optionally substituted, as indicated for alkyl and aryl.

The term "heterocyclic group" refers to and includes aromatic and non-aromatic cyclic radicals containing at least one, preferably 1 to 5, more preferably 1 to 3, most preferably 1 or 2 heteroatoms. Preferably the at least one heteroatom is selected from O, S, N, P, B, Si, and Se, more preferably O, S, or N. Hetero-aromatic cyclic radicals may be used interchangeably with heteroaryl. Preferred hetero-non-aromatic cyclic groups are those containing 3 to 7 ring atoms which includes at least one hetero atom, and includes cyclic amines such as morpholino, piperidino, pyrrolidino, and the like, and cyclic ethers/thio-ethers, such as tetrahydrofuran, tetrahydropyran, tetrahydrothiophene, and the like. Additionally, the heterocyclic group may be optionally substituted, e.g. by halogen, deuterium, alkyl or aryl. The heterocyclic group can be covalently linked with the remainder of the molecule via carbon and/or heteroatoms, preferably one carbon or nitrogen atom. The heterocyclic group can as well be linked with two carbon and/or heteroatoms with the remainder of the

molecules. In the case the heterocyclic group can be part of a cyclic group.

The term "aryl" refers to and includes both single-ring aromatic hydrocarbon groups and polycyclic aromatic ring systems. The polycyclic rings may have two or more rings in which two carbons are common to two adjoining rings (the rings are "fused") or wherein one carbon is common to two adjoining rings (e.g. biphenyl) wherein at least one of the rings is an aromatic hydrocarbon group, e.g., the other rings can be cycloalkyls, cycloalkenyls, aryl, heterocycles, and/or heteroaryls. Preferred aryl groups are those containing three to eight aromatic carbon atoms. Especially preferred is an aryl group having six carbons. Suitable aryl groups include phenyl, and radialene. Most preferred is phenyl that can be substituted by non-aromatic groups. Additionally, the aryl group is optionally substituted, e.g. by halogen, alkyl, hetero-alkyl, or deuterium. The aryl group is connected to the remainder of the ligand via one or two carbon atoms.

The term "heteroaryl" refers to and includes both single-ring aromatic groups and polycyclic aromatic ring systems that include at least one heteroatom. The heteroatoms include, but are not limited to and are preferably selected from O, S, N, P, B, Si, and Se. In many instances, O, S, or N are the preferred heteroatoms. Hetero-single ring aromatic systems are preferably single rings with 5 or 6 ring atoms, and the ring can have from one to five/six, preferably 1 to 3, more preferably 1 or 2 heteroatoms. The hetero-polycyclic ring systems can have two or more rings in which two atoms are common to two adjoining rings (the rings are "fused") or wherein one carbon is common to two adjoining rings (e.g. bipyridine) wherein at least one of the rings is a heteroaryl, e.g., the other rings can be cycloalkyls, cycloalkenyls. The hetero-polycyclic aromatic ring systems can have from 1 to 5, preferably 1 to 3, more preferably 1 or 2 heteroatoms per ring of the polycyclic aromatic ring system. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyrrole, pyrazole, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably pyridine, pyrrole, pyrazole, furan, carbazole, dibenzofuran or benzofuran groups, from which one hydrogen atom has been removed from a hydrogen-bearing carbon or heteroatom to form the covalent link to the remainder of the molecule. The heteroaryl group with two hydrogens removed can as well be linked with two carbon and/or heteroatoms with the remainder of the molecules, in which case the heteroaryl group can be part of a larger cyclic group. Additionally, the heteroaryl group is optionally substituted, e.g. by halogen, deuterium, alkyl or aryl.

Generally, aryl and heteroaryl groups with triplet level > 2.5 eV or more preferably >2.6 eV or most preferably >2.7 eV such as the groups derived from benzene, furan, dibenzofuran, dibenzoselenophene, carbazole, imidazole, pyridine, pyrazine, pyrimidine, triazine, and the respective aza-analogs of each thereof are of particular interest. There are many possible ways to probe the triplet energy level of an organic molecule. An easy way is to use suitable quantum mechanical calculations. A more direct way takes the onset of the phosphorescence spectrum, which is usually detected using gated spectroscopy, as pioneered by Romanovskii et al. "Phosphorescence of $\pi$-conjugated oligomers and polymers." Physical Review Letters 84.5 (2000): 1027. In combination with high triplet energy, generally preference is given to benzene or low polarizable, hard heteroaryl groups comprising oxygen only, such as furan, dibenzofuran, and benzofuran.

The alkyl, cycloalkyl, heteroalkyl, heterocycloalkyl, alkenyl, cycloalkenyl, heteroalkenyl, alkynyl, aralkyl, heterocyclic group, aryl, and heteroaryl groups or residues, as used herein, are independently unsubstituted, or independently substituted, with one or more (general) substituents, preferably the substituents mentioned below.

Any type of substituent can replace a hydrogen atom in an organic or heterorganic group of compound (a) or (b), as long as it results in a chemical compound which is stable under conditions that occur in an OLED display device or organic electronic device and which does not chemically react with other compounds and components of the OLED display device or organic electronic device.

Preferably, the (general) substituents are selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof with the number of carbon atoms and heteroatoms as defined above for the respective term. Furthermore, one or two substituents can be selected from polymer chains which can be covalently linked with the remainder of the molecule by a suitable organic spacer. Therefore, the cyclic organic ligand can be covalently linked with a polymer chain or a polymer backbone.

In some instances, the preferred general substituents are selected from the group consisting of deuterium, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof with the number of carbon atoms and heteroatoms as defined above for the respective term.

In yet other instances, the more preferred general substituents are selected from the group consisting of

deuterium, alkyl, cycloalkyl, aryl, heteroaryl, and combinations thereof with the number of carbon atoms and heteroatoms as defined above for the respective term.

The terms "substituted" and "substitution" refer to a substituent other than H that is bonded to the relevant position, e.g., a carbon or nitrogen. For example, when R represents mono-substitution, then one R must be other than H (i.e., a substitution). Similarly, when R represents di-substitution, then two of R must be other than H. Similarly, when R represents no substitution, R, for example, can be a hydrogen for available valencies of straight or branched chain or ring atoms, as in carbon atoms for benzene and the nitrogen atom in pyrrole, or simply represents nothing for ring atoms with fully filled valencies, e.g., the nitrogen atom in pyridine. The maximum number of substitutions possible in a straight or branched chain or ring structure will depend on the total number of available valencies in the ring atoms or number of hydrogen atoms that can be replaced. All residues and substituents are selected in a way that a chemically stable and accessible chemical group results.

As used herein, "combinations thereof" indicates that one or more members of the applicable list are combined to form a known or chemically stable arrangement that one of ordinary skill in the art can envision from the applicable list. For example, an alkyl and deuterium can be combined to form a partial or fully deuterated alkyl group; a halogen and alkyl can be combined to form a halogenated alkyl substituent; and a halogen, alkyl, and aryl can be combined to form a halogenated arylalkyl. In one instance, the term substitution includes a combination of two to four of the listed groups. In another instance, the term substitution includes a combination of two to three groups. In yet another instance, the term substitution includes a combination of two groups. Preferred combinations of substituent groups are those that contain up to fifty atoms that are not hydrogen or deuterium, or those which include up to forty atoms that are not hydrogen or deuterium, or those that include up to thirty atoms that are not hydrogen or deuterium counted for all substituents of a given molecule, or for the respective molecule in total. In many instances, a preferred combination of substituent groups will include up to twenty atoms that are not hydrogen or deuterium, counted for all substituents of a given molecule.

Polymacrocyclic or polycyclic are used interchangeable and refer to a molecule where two given atoms are at least three times linked with each other using at least partially aliphatic organyl groups. In this particular description the two threefold linked atoms are preferably nitrogen atoms, resulting in molecules known as cryptand ligands.

The "aza" designation in the fragments described herein, i.e. aza-cryptate, etc. means that one or more carbon atom or (other) heteroatom of a parent compound is replaced by a nitrogen atom, without any limitation. For example, in a crown ether - O- is replaced by -NH- to give the respective aza compound. One of ordinary skill in the art can readily envision other nitrogen analogs of the aza-derivatives, and all such analogs are intended to be encompassed by the terms as set forth herein.

As used herein, "deuterium" refers to an isotope of hydrogen. Deuterated compounds can be readily prepared using methods known in the art.

It is to be understood that when a molecular fragment is described as being a substituent or otherwise attached to another moiety, its name may be written as if it were a fragment (e.g. phenyl, phenylene, naphthyl, dibenzofuryl) or as if it were the whole molecule (e.g. benzene, naphthalene, dibenzofuran). As used herein, these different ways of designating a substituent or attached fragment are considered to be equivalent.

In some instances, a pair of adjacent or non-adjacent substituents or residues can be optionally joined (i.e. covalently linked with each other) or fused into a ring. The preferred ring formed therewith is a five-, six-, or seven-membered carbocyclic or heterocyclic ring, including both instances where the portion of the ring formed by the pair of substituents is saturated and where the portion of the ring formed by the pair of substituents is unsaturated. As used herein, "adjacent" means that the two substituents involved can be on the same ring next to each other, or on two neighboring rings having the two closest available substitutable positions, such as 2, 2' positions in a biphenyl, or 1, 8 position in a naphthalene, or 2,3-positions in a phenyl, or 1,2-positions in a piperidine, as long as they can form a stable fused ring system.

In this description, the term "organyl" refers to any chemically stable organic arrangement of atoms where one or more hydrogen atom has been removed such as to use those free vacancies to covalently link the organic group with another molecular entity. Thus, the term organyl encompasses the majority of the above defined organic groups e.g. fluorine, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof with the number of carbon atoms and heteroatoms as defined above for the respective term.

The term "substantially" may refer to (relative) amounts of a compound and typically means at least 80 wt%, preferably at least 90 wt%, more preferably at least 95 wt%, specifically 100 wt%, not considering impurities or additives. It may define ranges of 80 to 100 wt% etc. for the content or purity of a compound. This definition refers e.g. to amounts like weight or volume or other parameters like wavelength. For single values, "substantially" allows for a variation or deviation from a given number by 20% or less, preferably 10% or less, more preferably 5% or less, specifically 1% or less. An emission substantially in the deep blue spectral region below 500 nm means that at least 80% of the light emission integrated over wavelength are below 500 nm etc., e.g. of a measured integral of the emission intensity over wavelength. A substantially organic substrate contains at least 80% of organic substances. When not indicated otherwise, % means wt%.

Organic groups are derived or formed by removing one or more hydrogen atoms from organic molecules containing at least a corresponding number of hydrogen atoms. For example, benzene results in phenyl or phenylene groups by removing one or two hydrogen atoms, resulting in a monovalent or divalent organic group.

Suitable organic molecules and groups are as identified above, e.g. alkyl, aryl, heteroaryl groups. Sequences of atoms linking the two Ys are composed of atoms present in organic molecules, preferably of covalent carbon chains, in which one or more (preferably non-neighboring) carbon atoms can be replaced by heteroatoms. Preferably the (carbon) atoms covalently attached to the bridging nitrogen atoms are not replaced.

In this description, an "electroluminescent coordination compound" is any material that is able to emit light upon electrical excitation, followed by recombination of electrons and holes. It shall be irrelevant in this context whether the recombination of the electrons and holes takes place directly on the electroluminescent compound or first an excitation is formed on a different compound and subsequently transferred to the electroluminescent compound. Further, it shall be irrelevant in this context whether the light emitted is visible for the human eye or not. Especially, compounds emitting infrared and near ultra-violet emission shall be explicitly included. Further, the electroluminescent coordination compound does not necessarily have to be used in an electronic device but, for example, may be used as a dye or a contrast enhancement medium for magnetic resonance tomography.

The lanthanide included in the coordination compound according to the claims is Eu2+.

The macrocyclic organic ligand according to various embodiments may be combined with actinides. For example, the polycyclic organic ligand may be size discriminating for three-valent actinides, allowing their extraction from radioactive wastewater. Yet for application in electroluminescent devices lanthanides are preferred vs radioactive actinides.

In all embodiments, the coordination compound contains a polycyclic aza cryptand according to the generic formulas (1-1) and/or (1-2):

(1-1) (1-2)

with the above definitions of $\mathbf{L_0}$, $\mathbf{L_1}$, $\mathbf{L_2}$, **R.**

The two nitrogens together with the two covalently bonded linkers $\mathbf{L_1}$, and $\mathbf{L_2}$ form a basic cyclic di-aza-crown ether. According to the generic formulae (1-1) and (1-2) both nitrogens forming a cycle with $\mathbf{L_1}$ and $\mathbf{L_2}$ are further substituted in a characteristic manner, thereby linking with two oxygens via a spacer group containing two carbon atoms. Characteristically for (1-2), at least one of those two spacer carbon atoms as well the oxygen are part of a furan ring structure, whereby the furan is linked via the carbon 2-position. The polycyclic ligand according to formula (1-1) is completed by connecting those two oxygens with the linker $\mathbf{L_0}$. In case of formula (1-2) the polycyclic ligand is completed by connecting the furan groups with the carbon atom in the 5 position with the linker $\mathbf{L_0}$. All linkages between $\mathbf{L_0}$, $\mathbf{L_1}$, and $\mathbf{L_2}$ and the remainder of the organic ligand, respectively, are single bonds of aliphatic, non-aromatic nature to ensure high triplet energy of the polycyclic ligand according to the invention. Hetero-atoms in $\mathbf{L_0}$, $\mathbf{L_1}$ and $\mathbf{L_2}$ are preferably not in terminal positions in the shortest sequence of atoms.

(For explanation: If any L were 1,4-phenylene, it would have 4 atoms between the two linkages. In 1,3-phenylene it would have 3 atoms in the shortest sequence, and not 5.)

All linkages between $\mathbf{L_0}$, $\mathbf{L_1}$, and $\mathbf{L_2}$ and the two bridgehead atoms N are aliphatic single bonds (non-aromatic) to ensure high triplet energy of the polycyclic ligand according to the invention. $\mathbf{L_0}$, $\mathbf{L_1}$, and $\mathbf{L_2}$ in their further sequence of atoms may contain for example C-C double bonds or aromatic bonds as found in 1,4-, 1,3-or 1,2-phenylene groups, i.e. they include one or more aromatic ring structures as shown below.

In a preferred embodiment, the organyl groups $\mathbf{L_0}$, $\mathbf{L_1}$, and $\mathbf{L_2}$ present in (1-1) and (1-2) are comprising only of hydrogen, and/or deuterium, carbon, and oxygen. In this preferred embodiment $\mathbf{L_1}$, and $\mathbf{L_2}$ preferably contain at least one oxygen such as present in ether, furan, benzofuran, or tetrahydrofuran fragments.

For chemical stability reasons, general preference is given to symmetrical linkers $\mathbf{L_1}$ and $\mathbf{L_2}$ such that the shortest sequence of $\mathbf{L_1}$ and $\mathbf{L_2}$ between the two bridgehead nitrogens is exactly 5 atoms and the heteroatom present in $\mathbf{L_1}$ and $\mathbf{L_2}$ is in the centre of this shortest sequence. Each of $\mathbf{L_1}$ and $\mathbf{L_2}$ then has the same linking groups on both sides of the heteroatom, leading to the symmetry within $\mathbf{L_1}$ and $\mathbf{L_2}$, respectively. Thus, the preferred embodiment for the shortest sequences of $\mathbf{L_1}$ and $\mathbf{L_2}$ between the two bridgehead nitrogens is of the type - C- C- X- C- C-, with X being the heteroatom O, with C being a substituted or non-substituted carbon atom, whereby the term substitution as well includes being part of an aryl heteroaryl

or otherwise cyclic ring system. In general, preference is given to embodiments with $\mathbf{L_1}$ and $\mathbf{L_2}$ being equal/the same.

Examples of polycyclic ligands according to formula (1-1) and (1-2) with $\mathbf{L_1}$ and $\mathbf{L_2}$ being equal and mirror symmetrical with respect to the shortest sequence between the two bridgehead nitrogen in (1-1) and (1-2) utilizing ethyleneoxy, or dimethylfuran fragments as the shortest sequences of $\mathbf{L_1}$ and $\mathbf{L_2}$ between the two nitrogen atoms and derived from the generic formula (1-1) by specifying $\mathbf{L_1}$ and $\mathbf{L_2}$ are given in (1-3) and (1-5), whilst (1-6) and (1-8) are derived from (1-2)

1-3

1-5

1-6

1-8

The preferred organyl group is in both instances a methyl group. Here, the shortest sequence between the two nitrogen bridge head atoms is 5 atoms whereby $\mathbf{L_1}$ is equal to $\mathbf{L_2}$ and the at least one heteroatom present in both $\mathbf{L_1}$ and $\mathbf{L_2}$ is selected from the preferred candidates nitrogen and oxygen such that the linkers comprise ethyleneoxyor dimethylfuran fragments.

Within the scope of this invention, a wide range of linkers $\mathbf{L_0}$ is chemically easily accessible, preferably chosen from substituted or non-substituted alkane, arene, or heteroarene, formed by removing two hydrogen atoms therefrom, preferably allowing for the shortest sequence of preferably not more than 7 atoms, more preferably of 3 to 7 , most preferably of 3 or 5. In order to ensure stable complex formation as part of the metal-organic coordination compound, the preferred linker $\mathbf{L_0}$ has a shortest sequence of not more than 7 atoms. Similar to allow for more stable symmetrical configurations, the preferred linker has an odd number of atoms in its shortest sequence. For $\mathbf{L_0}$ as part of formula (1-1), the even more preferred number of atoms as shortest sequence between the remainder of the macrocyclic ligand is 5 or 3, while the most preferred length of the sequence is 5 atoms. For $\mathbf{L_0}$ as part of formula (1-2), the most preferred number of atoms as shortest sequence between the remainder of the macrocyclic ligand is 3. $\mathbf{L_0}$ may comprise one or more hetero atoms able to complex and therefore stabilize the metal cation to be coordinated. Preferably, $\mathbf{L_0}$ is thus a divalent organic group formed by removing two hydrogen atoms from an organic molecule containing at least two hydrogen atoms, with the shortest sequence of atoms in $\mathbf{L_0}$ linking the two oxygen atoms in (1-1) and carbon atoms in (1-2) adjacent to $\mathbf{L_0}$ being each 7 or less, preferably each 7, 5 or 3 carbon atoms long, wherein one non-terminal carbon atom of these can be replaced by B, N, P, S or O, preferably N or O, more preferably O.

Further preferred polycyclic ligands in view of the above preferences may be selected from (1-9) to (1-21):

1-9
1-10
1-11
1-12
1-13
1-14
1-15
1-16
1-17
1-18
1-19
1-20
1-21

With $R_b$ independently in each occurrence representing hydrogen, deuterium, halogene - preferably chlorine, - or, straight or branched alkyl chain radicals including cycloalkyl radicals, the alkyl groups containing from one to fifteen, preferably one to ten, more preferably one to five carbon atoms, the term "cycloalkyl radical" referring to monocyclic, polycyclic, and spiro alkyl radicals containing 3 to 12, preferably 3 to 8 ring carbon atoms , and n independently in each occurrence being an integer in the range of 0 to 10, preferably 1 to 3. In those examples ethyleneoxy or dimethylfuran have been chosen for both $\mathbf{L_1}$ and $\mathbf{L_2}$. Additionally, the shortest sequence of $\mathbf{L_0}$ between the remainder of the polycyclic ligand according to formulae (1-1) and (1-2) is chosen to be 5 and 3, respectively, with a single oxygen being in the center of this sequence such that the linker becomes symmetrical too. $\mathbf{L_0}$ may be a simple (di-ethylene)oxy or (di-methyl)oxy chain, such as in 1-10, 1-13, 1-14, 1-16, 1-19, 1-20, or 1-21. Alternatively, $\mathbf{L_0}$ may be a di-benzofuran or di-methylfuran with a shortest sequence of 5 atoms derived from the generic formula (1-1) as exemplified in (1-11) and (1-18) or (1-9) and (1-15), respectively. The majority of the carbon atoms present in the generic formulas (1-9) to (1-21) may be substituted, if they are linked to a hydrogen atom, however, for clarity reasons most of those optional groups have been omitted in the shown formulas, which, however, shall not limit the scope of this invention. Indeed, suitable substitutions, such as deuterium or short, preferably $C_{1-3}$-, e.g. methyl or ethyl alkyl groups may be beneficial for thermal stability or improve the solubility and/or volatility. For chemical stability reasons and to avoid unnecessary polarization, preference is generally given to symmetrical substitution pattern with

respect to the two bridge head nitrogen atoms. For example, in (1-9) the two Rs shown would be preferably equal/identical. Similarly, if anionic negative side groups are attached to the ligands according to the generic formulas (1-1) and (1-2), then preferably two such groups are attached symmetrically; and preferably low polarizing groups that do not absorb light in the visible range are chosen. Suitable covalent anionic substitution are found in the class of closo-borane or *closo*-carborane clusters and examples for their symmetrical attachment toward the remainder of the ligand according to formula (1-1) are given in (1-16), (1-19), and (1-21).

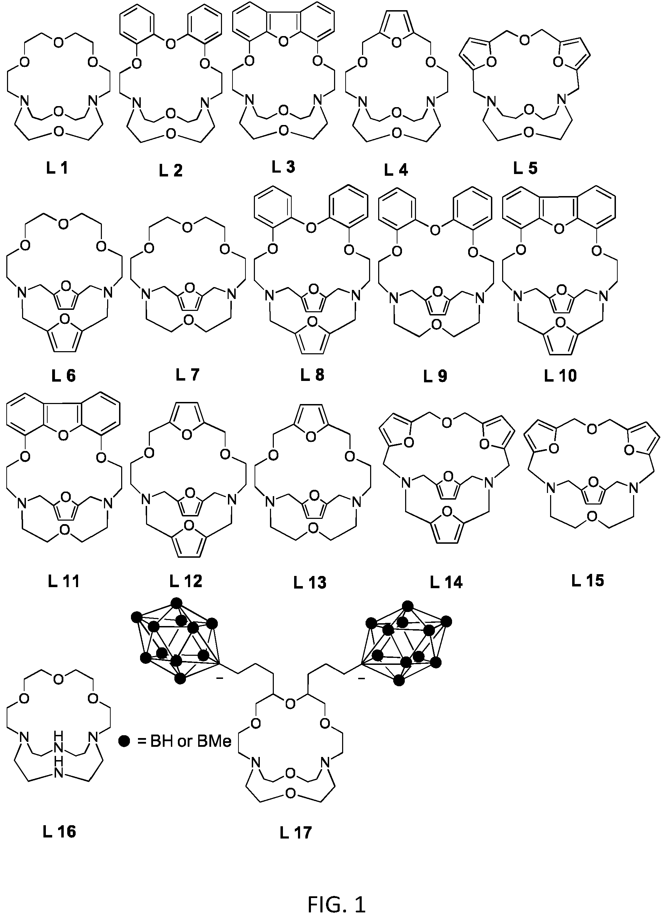

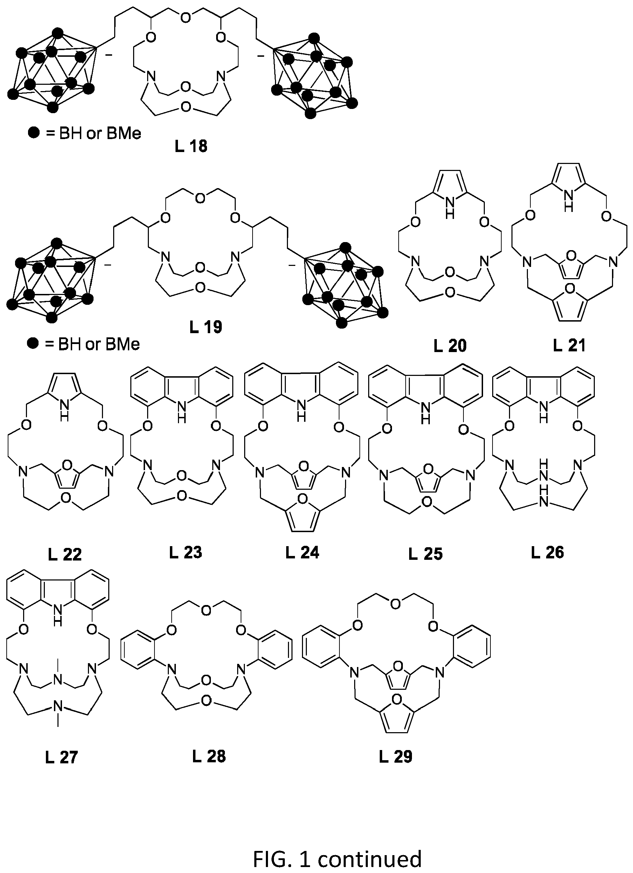

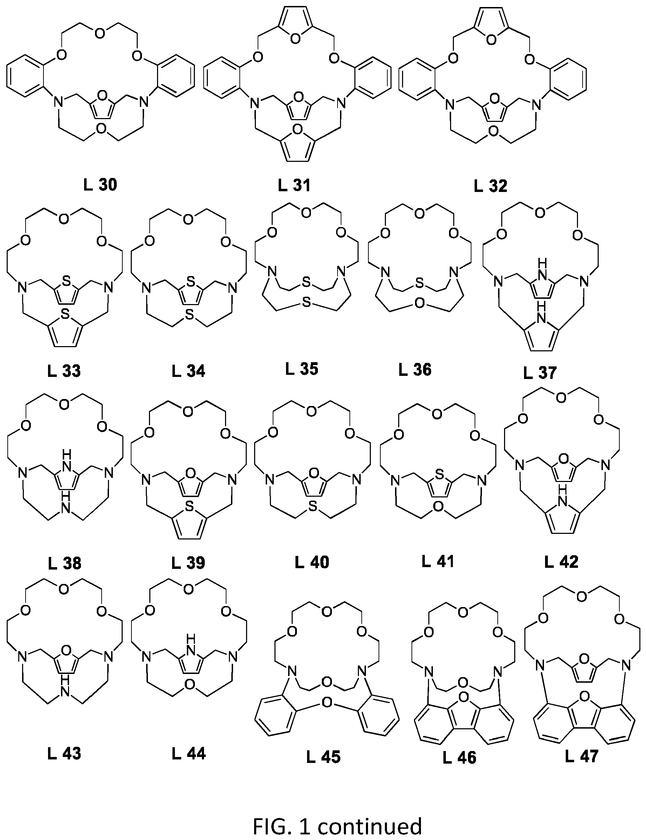

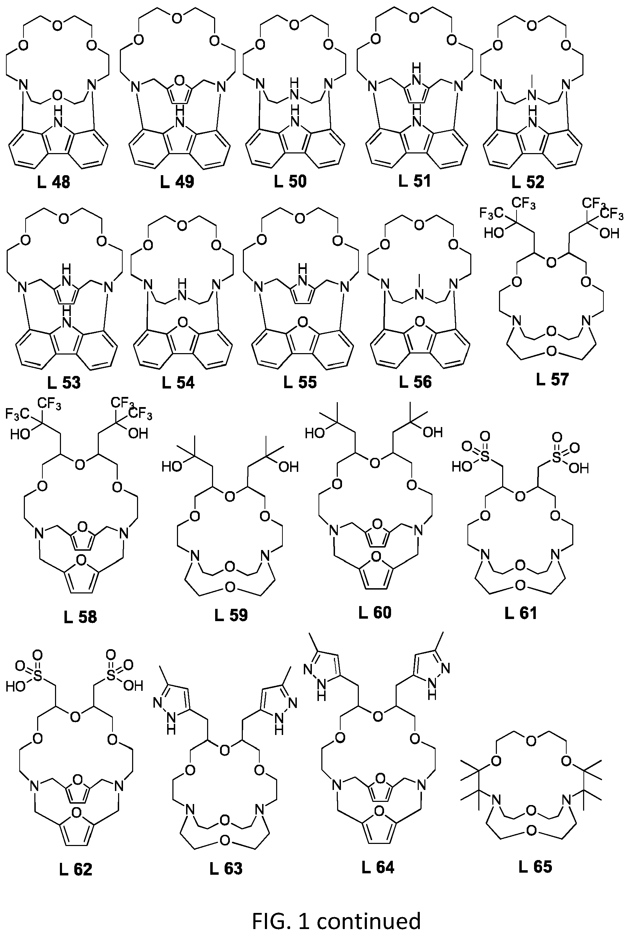

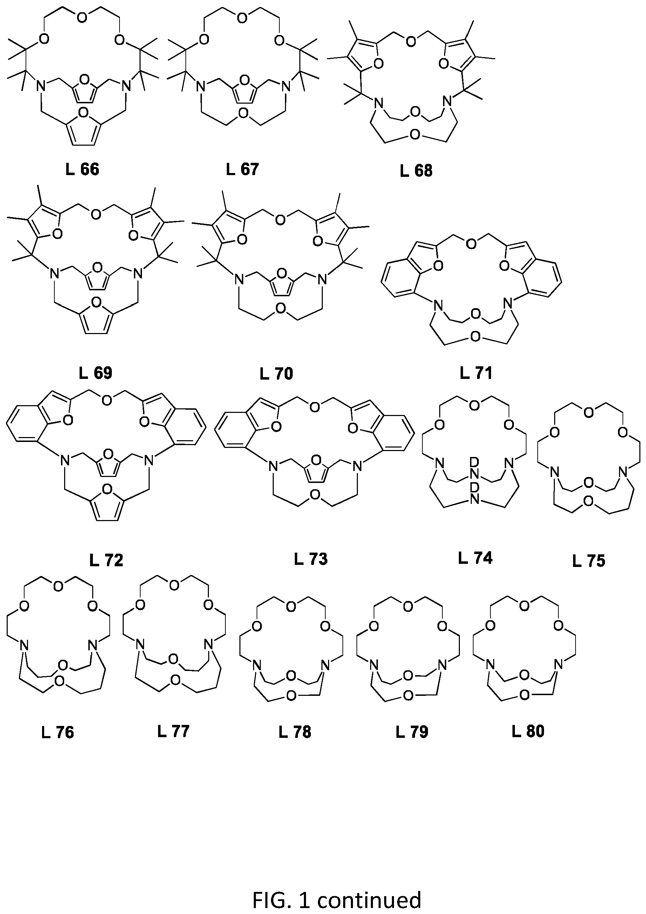

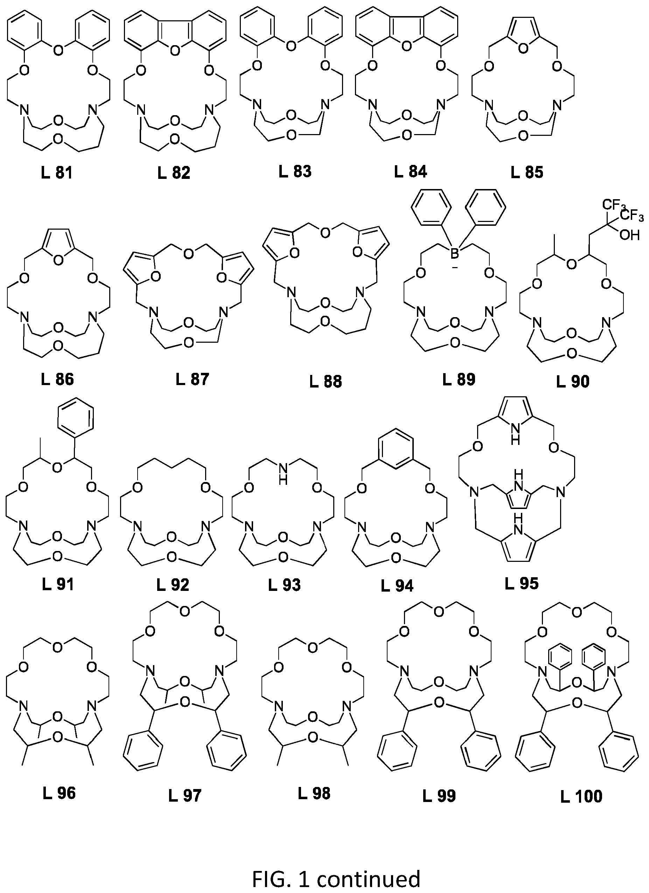

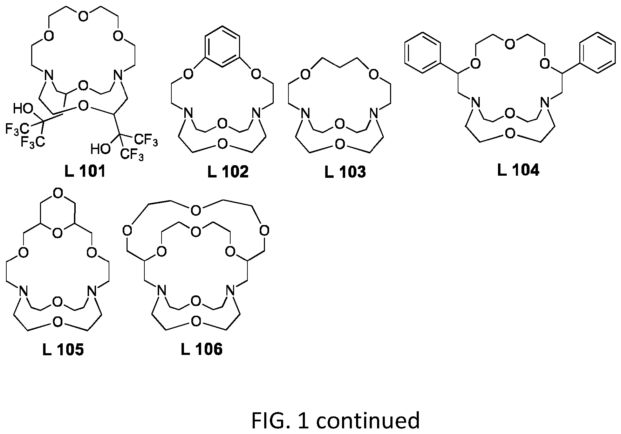

**FIG. 1** further illustrates examples of the organic ligand according to various embodiments without limitation and represented by the generic formulae (1-1) and (1-2) with linkers, $\mathbf{L_1}$, $\mathbf{L_2}$, and $\mathbf{L_0}$.

In the invention examples depicted in **FIG. 1** the shortest sequence of the two linkers $\mathbf{L_1}$, and $\mathbf{L_2}$ is 5 atoms and the one hetero atom is in the center of this 5 atom sequence. Reference examples not according to the present invention with different shortest sequence of $\mathbf{L_1}$, and $\mathbf{L_2}$ are L75-L88 where either $\mathbf{L_1}$ or $\mathbf{L_1}$, and $\mathbf{L_2}$ possess of 4 or 6 atoms. For example, in L75, L81, and L82 the shortest sequence of $\mathbf{L_1}$ is 5 atoms while it is 6 atoms for $\mathbf{L_2}$. Similarly, in L78, L83, L84, and L85 the shortest sequence of $\mathbf{L_1}$ is 5 atoms while it is 4 atoms for $\mathbf{L_2}$. L76, L77 and L79, L80 illustrate the situation of both $\mathbf{L_1}$, and $\mathbf{L_2}$ being 6 and 4 atoms, respectively, in their shortest sequence of atoms between the two bridge head nitrogens. Indeed, such modified length of $\mathbf{L_1}$, and $\mathbf{L_2}$ do alter the diameter of the basis aza-crownether, which may be beneficial to adapt the cavity of the polycyclic ligand according to formulae (1-1) or (1-2) perfectly to a certain given lanthanide central cation.

In the invention examples depicted in **FIG. 1** the one heteroatom that is present in the shortest sequence of the two linkers $\mathbf{L_1}$, and $\mathbf{L_2}$ is oxygen. Other heteroatoms are possible in reference examples which are not according to the present invention, such as S, B, P. For example, reference L33-L36 and L39-L41 illustrate the integration of a 5 atom shortest sequence of a diethylthio fragment in either $\mathbf{L_1}$ or $\mathbf{L_1}$ and $\mathbf{L_2}$. The heteroatom present in $\mathbf{L_1}$ and $\mathbf{L_2}$ is the same,yet different heteroatoms in both linkers are possible for reference examples only, which may for example be easier synthetically accessible. For example in reference L36, L40, L41 with O and S two different heteroatoms are present in $\mathbf{L_1}$ and $\mathbf{L_2}$ whilst reference L42-L44 illustrate the same situation for the heteroatoms O and N.

Whilst $\mathbf{L_1}$, and $\mathbf{L_2}$ must contain a heteroatom each in their shortest sequence between the two bridge head nitrogen atoms, $\mathbf{L_0}$ may contain one or more heteroatoms. For example L92, L94, L102, L103 based on the generic formula (1-1) depict the situation of $\mathbf{L_0}$ being just 3 or 5 carbon atoms, without any heteroatom. However, preference is given to macrocyclic ligands according to formulae (1-1) and (1-2) with $\mathbf{L_0}$ comprising heteroatoms in the shortest sequence of atoms linking $\mathbf{L_0}$ to the remainder of the macrocyclic ligands in (1-1) and (1-2). Preferably only one heteroatom is present, and this preferably one heteroatom is selected from B, N, P, S, O, preferably form N, O, and even more preferably the one heteroatom is oxygen. Preferably, also $\mathbf{L_0}$ is symmetrical, as outlined above for $\mathbf{L_1}$ and $\mathbf{L_2}$.

In various embodiments of this invention the linker $\mathbf{L_1}$, $\mathbf{L_2}$, and $\mathbf{L_0}$ present in (1-1) and (1-2) only comprise of hydrogen, and/or deuterium, carbon, and oxygen. This situation is for example illustrated in examples L1-L15, L19, or L65-L73 in **Fig 1.**

In various embodiments the metal-organic coordination compound comprising the ligands according to formulae (1-1) or (1-2) are used to generate deep blue light with high emission yield. This is best achieved if the excitation is confined on the central metal, thereby avoiding significant formation of charge transfer states or energy transfer between the central metal and the coordinating ligand according to formulae (1-1) and (1-2). To ensure energy confinement on the metal central atom, the ligand should have a triplet energy higher than the emission energy of the intrametallic transition. Consequently, the triplet energy of the ligand must be at least >2.5 eV, preferably >2.6 eV and even more preferably >2.7 eV in case the complex is intended to emit in the deep blue spectral region. Triplet energies are measured e.g. as described in Romanovskii, Yu V., et al. "Phosphorescence of $\pi$-conjugated oligomers and polymers." Physical Review Letters 84.5 (2000): 1027. Consequently, the buildings blocks of the polycyclic organic ligand according to formulae (1-1) and (1-2) are preferably either non-aromatic (aliphatic) or comprise aromatic building blocks containing at most 8 aromatic carbon or hetero atoms, whereby preferably aromatic building blocks are being separated from each other by at least one nonaromatic carbon or heteroatom.

In various embodiments of this invention at least two atoms present in the shortest sequence of atoms of either $\mathbf{L_0}$, $\mathbf{L_1}$, or $\mathbf{L_2}$ are part of a benzene, pyridine, pyrrole, pyrazole, furan, carbazole, dibenzofuran, benzimidazole, or benzofuran ring structure, whereby preference is given to furan. For example in L94 and L102, two carbon atoms present in the shortest sequence of $\mathbf{L_0}$ are fused into a benzene ring. Similarly, in L2, L8, L9, L81, and L83, four carbon atoms present in the shortest sequence of $\mathbf{L_0}$ are part of two non-fused benzene rings. In L3, L10, L11 and in L23-L27 each of the 5 atoms present in the shortest sequence of $\mathbf{L_0}$ are part of a dibenzofuran and carbazole ring structure, respectively. However, preference is given to furan aromatic ring structures, which is exemplified for the case of introduction into $\mathbf{L_0}$ in structures L4, L12, L13, L31, L32, L86 and for the case of introduction into either $\mathbf{L_1}$, or $\mathbf{L_2}$ in structures L7, L9, L11, L15, L22, L30, L32, L40, and L43 and so on. In even more preferred situations, both $\mathbf{L_1}$, and $\mathbf{L_2}$ comprise a furan unit as part of their shortest sequence of atoms, which is exemplified in Fig 1 in L6, L8, L10, L12, L14, L21, L24, L29, and so on. Furan, benzene, and pyridine contain less than 8 aromatic carbons or heteroatoms. Dibenzofuran, carbazole can be considered as two benzene rings linked by non-aromatic hetero or carbon atoms according to the definition above, and thus ensure sufficiently high triplet energy of the ligand system. "Shortest sequence" shall always be the shortest sequence (i.e.

number) of atoms that link the two bridge head nitrogens in case of $\mathbf{L_1}$, or $\mathbf{L_2}$ or that link $\mathbf{L_0}$ with the remainder of the macrocyclic ligand according to formulae (1-1) or (1-2). By example of L102 and L103, in both cases the shortest sequence of $\mathbf{L_0}$ is 3 atoms, whilst for L74-L86 the shortest sequence of $\mathbf{L_0}$ is 5 atoms.

While the heteroarene groups may be introduced into the shortest sequence of $\mathbf{L_1}$, $\mathbf{L_0}$, or $\mathbf{L_2}$ in various ways, general preference is given to connecting them symmetrically relative to the one heteroatom or heteroatoms. Further and preferentially, heteroaromatic groups are introduced into the shortest sequence of $\mathbf{L_1}$, $\mathbf{L_0}$, or $\mathbf{L_2}$ in a way that the heteroatom itself is part of the shortest sequence of $\mathbf{L_1}$, $\mathbf{L_0}$, or $\mathbf{L_2}$. Thus, for example in the case of the five membered heteroaryl groups furan, thiophen, or pyrazole the preferred connecting carbon atoms are the 2 and 5 positions neighbouring the heteroatom, while for carbazole and dibenzofuran the preferred connection points are the 1 and 8 positions as illustrated in examples L46 - L56 in FIG. 1.

The coordination compound according to various embodiments may contain a ligand according to formulae (1-1) or (1-2) with a plurality of substituents at positions **R,** which are not equal to hydrogen. Additionally, the organyl groups represented by $\mathbf{L_1}$, $\mathbf{L_2}$, or $\mathbf{L_0}$ may contain carbon or heteroatoms suitable for bearing further substituents. For example, in the generic formula (1-7) nitrogens present in $\mathbf{L_1}$ and $\mathbf{L_2}$ are optionally substituted, preferably with an alkyl group. Similarly, carbon atoms present in $\mathbf{L_0}$ of the generic formulae (1-9), (1-11), (1-12), or (1-16) are substituted in a way that in the first three examples aryl or heteroaryl groups are formed and in (1-16) two closocarborane clusters are linked to the remainder of the ligand according to formula (1-1) using an alkane chain linker of variable length n. As exemplified in (1-9), the substitution itself - in this particular case forming a furan ring - may be further substituted. In a similar logic, the generic formulae (1-5) and (1-8) being specific examples of (1-1) and (1-2), respectively, are formed by a substitution in $\mathbf{L_1}$ and $\mathbf{L_2}$ whereby two carbon atoms in each linker are substituted thereby forming a furan ring.

Preferably 0 to 20, more preferably 0 to 10 of these substituents can be present. For example, in (1-20) none of the suitable atoms is substituted. Yet in (1-11) and (1-12) 8 carbon atoms are substituted.

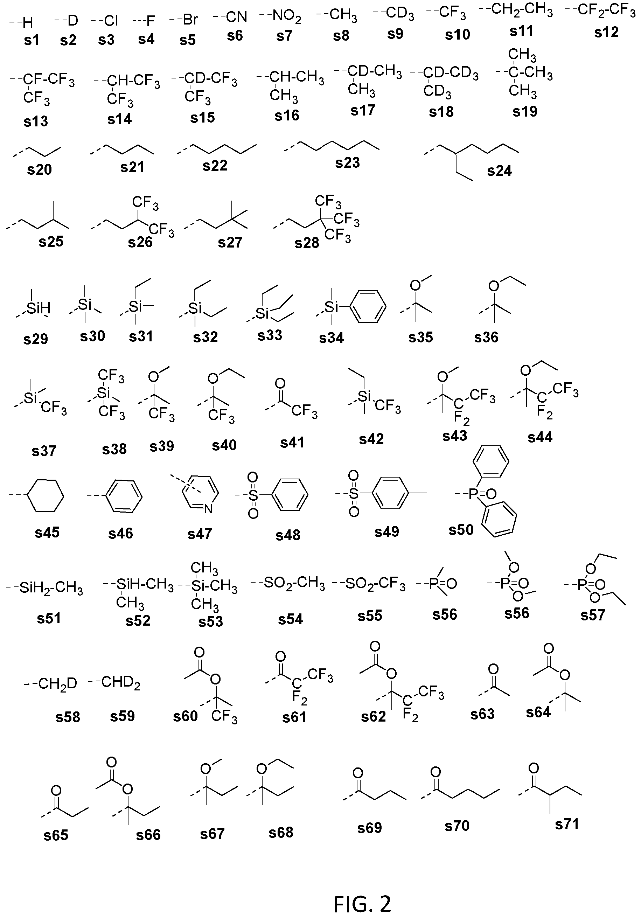

In general, substituents may be any chemical fragment that can be covalently attached in accordance with formulae (1-1) or (1-2). Examples of suitable substituents s2 to s71 are illustrated in **FIG. 2** wherein the dashed line indicates the single covalent link of the substituent with the main part of the ligand. Alternatively, two or more carbon atoms may simultaneously be substituted by a single substitute, whereby the two or more substituted carbon atoms may become part of an arene ring structure. In the generic formulae of the ligands (1-1) or (1-2), **R** independently in each occurrence represent hydrogen, or deuterium, or halogen, or an organyl group. Therefore a wide variety of substitution patterns can be considered and may be preferable in various embodiments of this invention. In **FIG. 1** often potential substitutions, **R,** are set to hydrogen for sake of clarity of the drawing. This, however, shall not limit the general scope of the invention, as any chemical fragment that can be covalently attached is in the scope of the invention. Examples where some of the possible substituents are explicitly specified include L27, L67, and L59, where alkyl and heteroalkyl chains are covalently attached. In L65-L70, some of the carbon atoms present in the scaffold of the ligand are twofold substituted. In L74, two hydrogens are substituted by deuterium. In L2 or L32, two neighboring carbon atoms present in $\mathbf{L_0}$ are substituted to form benzene rings, whereas in L4 and L20 two non-neighboring carbon atoms are substituted to form a furan and pyrrole heteroaryl group, respectively. Besides such a fusion of two or more carbon atoms into aryl or heteroaryl groups, it is as well possible that two substituents are linked with each other to form a non-aromatic cyclic group, for example becoming part of a cyclohexane cyclus. To further exemplify such embodiments, L105 and L106 illustrate cases where two R are linked together to form a hetero alkane. Generally, substitution patterns may reduce the symmetry of the polycyclic ligand relative to the unsubstituted base structure. For example, in L15 and L50, $\mathbf{L_1}$ is differently substituted from $\mathbf{L_2}$. Similarly, in L90 and L91, $\mathbf{L_0}$ is substituted twice with different substituents and therefore the symmetry of this linker is reduced. While substitution patterns that reduce the symmetry of the ligand are clearly within the scope of the invention, they are not the preferred options as often asymmetry lowers the chemical stability of the molecules and/or induces undesired polarizability during optical excitation or increases the dipole moment of the molecule. On the other hand side, asymmetrical substitution patterns may improve the solubility of the final coordination compounds or reduce their tendency to crystalize.

As said above, substitutions are by no means limited to the linkers $\mathbf{L_1}$, $\mathbf{L_0}$, or $\mathbf{L_2}$ as any other carbon or heteroatom present in the scaffolding of the ligands according to formula (1-1) or (1-2) may as well be substituted. Examples of this situation are L65-L73, L104, and L106 in Fig. 1. As explained above, preference is given to building blocks of the ligand according to formula (1-1) and (1-2) with sufficiently high triplet energy, ideally above 2.7eV. In the same logic as above, preference is given to aliphatic side groups or to aromatic side groups with less than 8 aromatic carbon or heteroatoms. Examples of such preferred aromatic substitution patterns are illustrated in L64, L73, and L99 in Fig. 1.

Examples of charge neutral substituents are s2 to s71 illustrated in **FIG. 2,** whereby a dashed line indicates the preferred covalent link of the shown molecular fragment to the carbon atoms of the polycyclic ligand according to formulae (1-1) and (1-2). Those fragments are meant as illustration for the scope of the invention only. Many more side groups may be chosen, depending on the final application. Easily envisioned for those skilled in the art are charge neutral substituents, for example alkane or alkene fragments, with two or more covalent links to two or more carbon atoms of the polycyclic ligands according to the invention. In other words, not shown are substitutes suitable to link two or more carbon atoms present in the ligands according to the invention. Otherwise, for example, linear or branched alkane side chains may be

chosen if a good solubility in non-polar solvents is needed. For other applications, coordination compounds with high thermal stability, for example suitable for evaporation or sublimation, may be needed. In this case, small and light weight substituents are preferably employed, i.e organyl fragments with individually less than 20 atoms and or individually a molecular weight of less than 100 g/mol. Similarly, halogenated, particularly fluorinated, side groups, such as depicted in L57 or L58 in **Fig 1** may improve volatility and/or stability of the coordination compound according to the invention. In case the intended application of the coordination compound of the invention is light generation, for example by electroluminescence, the ligand preferably may be chosen such that the excitation of the coordination compound is confined to the central lanthanide cation. In this way, an intrametallic transition with highest efficiency is obtained. With respect to the choice of the substituents, for example in the positions R in (1-1) or (1-2), preferred are side groups that are not easily polarized and that have a sufficiently high triplet level. Low polarizability is ensured for aliphatic or heteroaliphatic substituents or using small aryl- or heteroaryl systems with less than 8 conjugated atoms or heteroatoms. In case heteroatoms are part of conjugated side groups, preference is generally given to the heteroatoms N and O, and even more preference is given to O. The term "sufficiently high triplet energy" depends on the emission color of the complex. For example, if the intended emission color is in the near infrared regime, for example by using $Er3+$ as the central lanthanide, side groups comprising extended aryl, or heteroaryl organyl groups, such as phenanthroline may still offer sufficiently high triplet levels to ensure confinement of the excitation on the metal cation. On the other hand, if the coordination compound is intended to emit in the visible and in particular in the deep blue spectral region, then side groups with a triplet energy >2.5 eV, or preferably >2.7 eV should be used. Sufficiently high triplet energy >2.7 eV is generally observed for organyl groups comprising of aliphatic or heteroaliphatic fragments or for fragments comprising only small aryl- or heteroaryl systems with less than 8 conjugated atoms or heteroatoms, which thus should preferably be employed substituents if a deep blue electroluminescent emitter is the intended use of the coordination compound according to the invention. Examples of suitable side groups with respect to their triplet energy level are s2 to s71 illustrated in **FIG. 2.**

In various embodiments, a compound may include the coordination compound according to any of the described or illustrated embodiments and a polymer with a molecular weight $M_n$ above 1000 g/mol. In various embodiments, the coordination compound may be covalently attached to the polymer backbone for example via the side groups **R** in formulae (1-1) or (1-2). Alternatively, the covalent link may be formed using a suitable linker covalently attached to $\mathbf{L_0}$, $\mathbf{L_1}$, or $\mathbf{L_2}$. Thus, one of the **R** groups in (1-1) and (1-2) can be a polymer ($M_n$ > 1000 g/mol) having one free valence, formed by removing a hydrogen atom from the polymer. For example, in any of the positions R in formulae (1-7), (1-9), or (1-15) the linking with the polymer can be achieved.

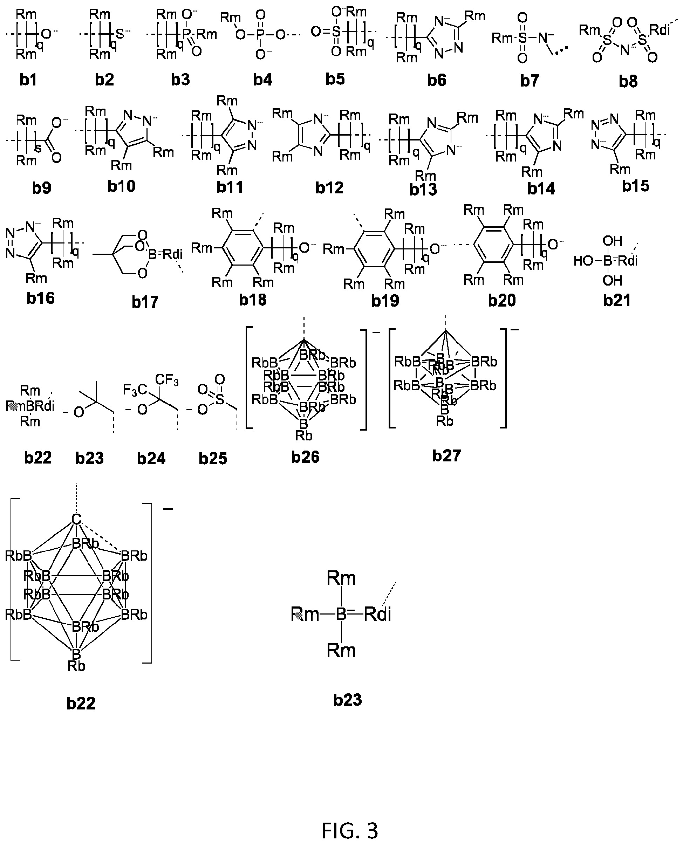

In various embodiments, the polycyclic organic ligand according to formulae (1-1) or (1-2) that coordinates to a lanthanide cation within the coordination compound according to invention may itself already be single or multiple negatively charged. In various embodiments, suitable anionic groups are covalently attached to the polycyclic organic ligand according to formulae (1-1) or (1-2) at positions **R.** Examples of such mono-anionic substitutions are illustrated in b1-b27 in **FIG. 3,** wherein the dashed lines indicate the linkage to the remainder of the ligand according to formulae (1-1) or (1-2).Therein, $\mathbf{R_b}$ independently in each occurrence represents either hydrogen, halogen, or a alkyl, preferably methyl and $\mathbf{R_m}$ independently in each occurrence represents monovalent groups formed by removing a hydrogen atom from a substituted or non-substituted alkane, or arene, or heteroarene, and $\mathbf{R_{di}}$ represents a divalent group formed by removing two hydrogen atoms from a substituted or non-substituted alkane, or arene, or heteroarene, and **q** independently in each occurrence is 0, 1, 2, or 3 and **s** independently in each occurrence is 1, 2, or 3.

Examples of twofold negatively charged polycyclic ligands according to formula (1-1) include L17-L19 in **Fig.1** with two substituted or non-substituted singly negatively charged closo-carborane clusters are being covalently attached to the remainder of the macrocyclic ligand via a carbon atom. Similarly, examples L57-L64 in Fig 1 illustrate a plurality of possible anionic groups being covalently attached to the remainder of the macrocylic ligand according to formula (1-1). In those examples, the hydrogenated neutral version of the polycyclic ligand is depicted. However, it is understood that a twofold negatively charged ligand is easily obtained after deprotonation of the side groups. L90 represents an example of a singly negatively charged ligand comprising covalently attached fluorinated and branched alcohol becoming an anionic alcoholate after deprotonation.

Instead of covalently attaching anionic groups, the polycyclic organic ligand may as well comprise negatively charged hetero atoms, such as borates or negatively charged aryl groups, such as pyrrolyl, as part of the scaffold of the polycyclic organic ligand. For example this can be achieved by deprotonation of the aromatic pyrrole building blocks depicted as reference L48-L53 in **FIG. 1.** The optional negative charge present in the ligand may as well be substantially localized on a particular heteroatom that is part of the scaffold of the organic ligand according to the invention that is generically represented by formulae (1-1) or (1-2). For example in **Fig. 1** L 89 a negatively charged borate is part of the linker $\mathbf{L_0}$, illustrating a singly negatively charged ligand according to formula (1-1). Preferably, the number of covalently (or non-covalently) attached negative charges equals the oxidation state of the lanthanide, so that a charge-neutral complex results.

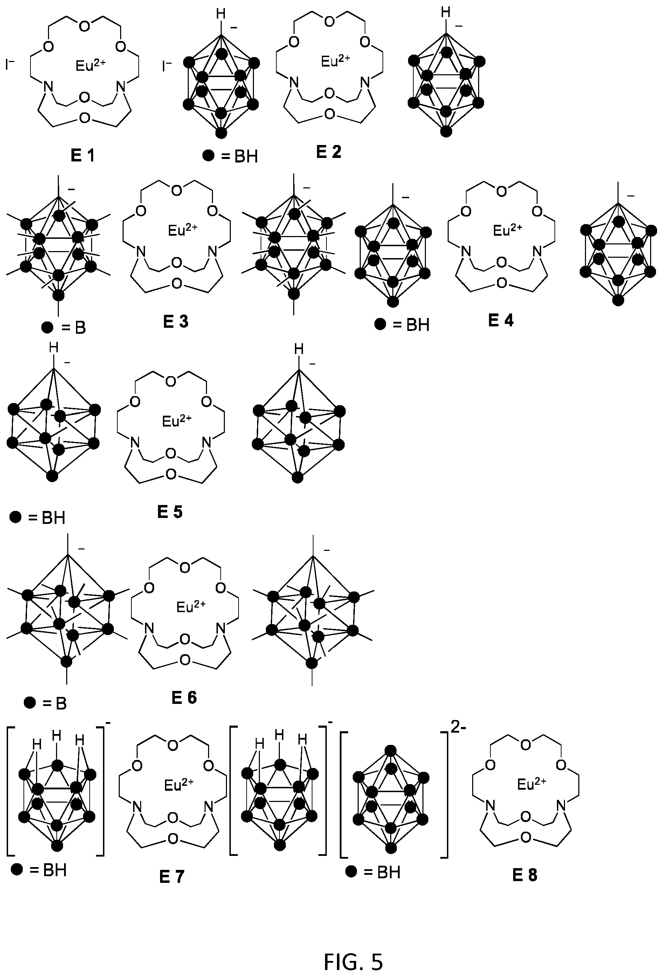

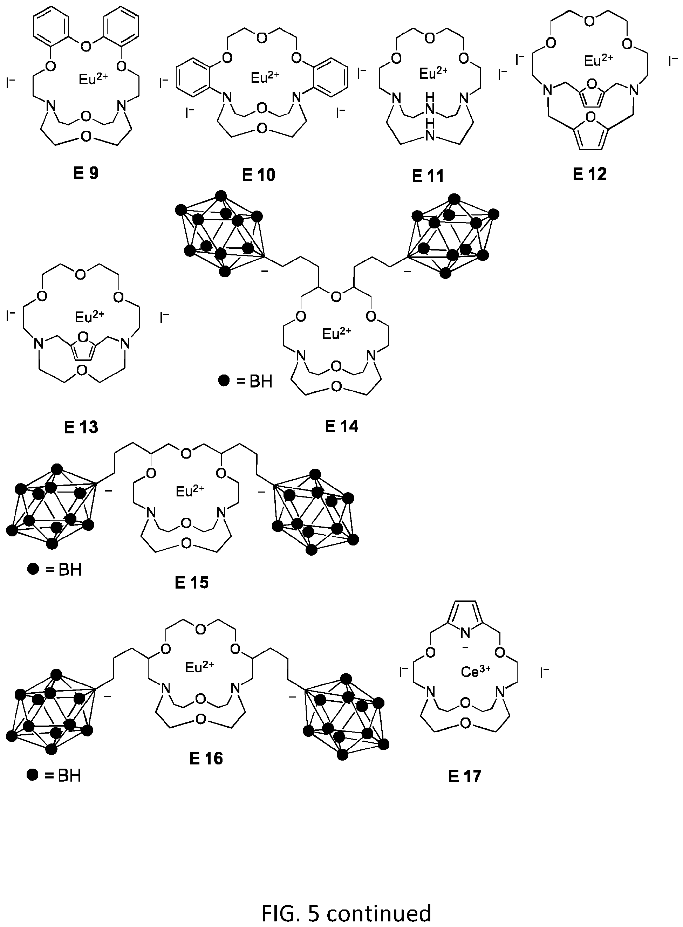

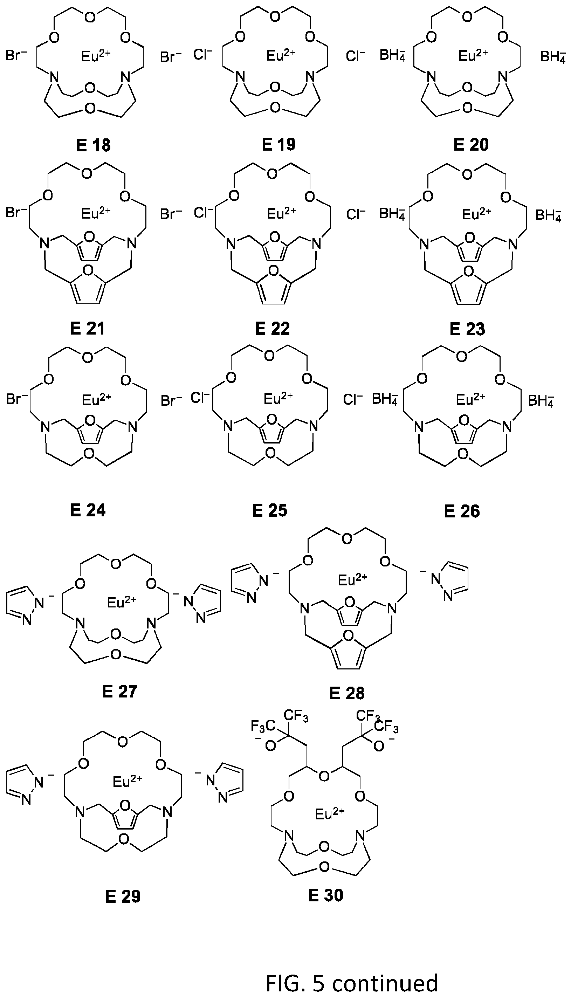

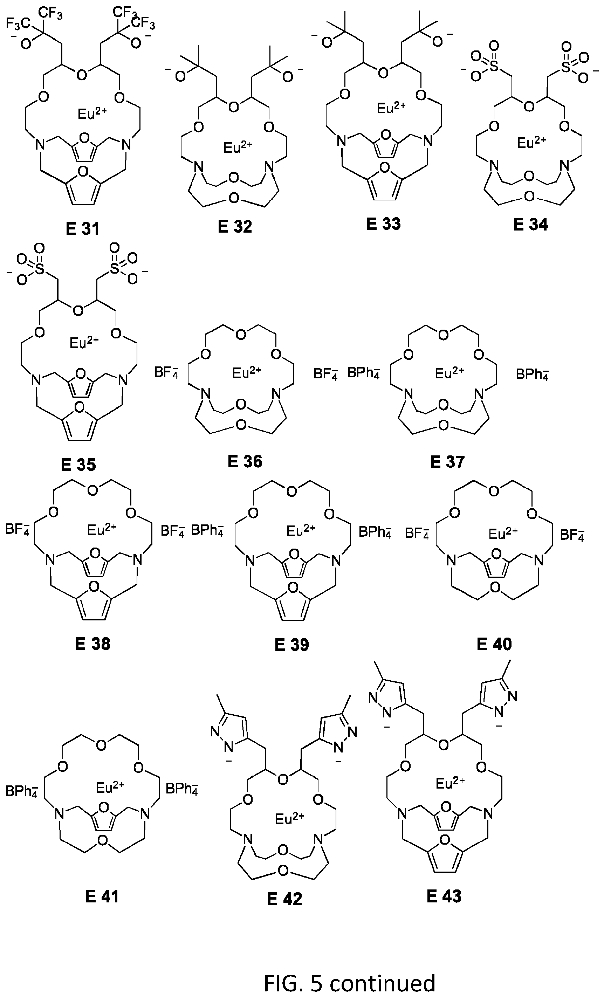

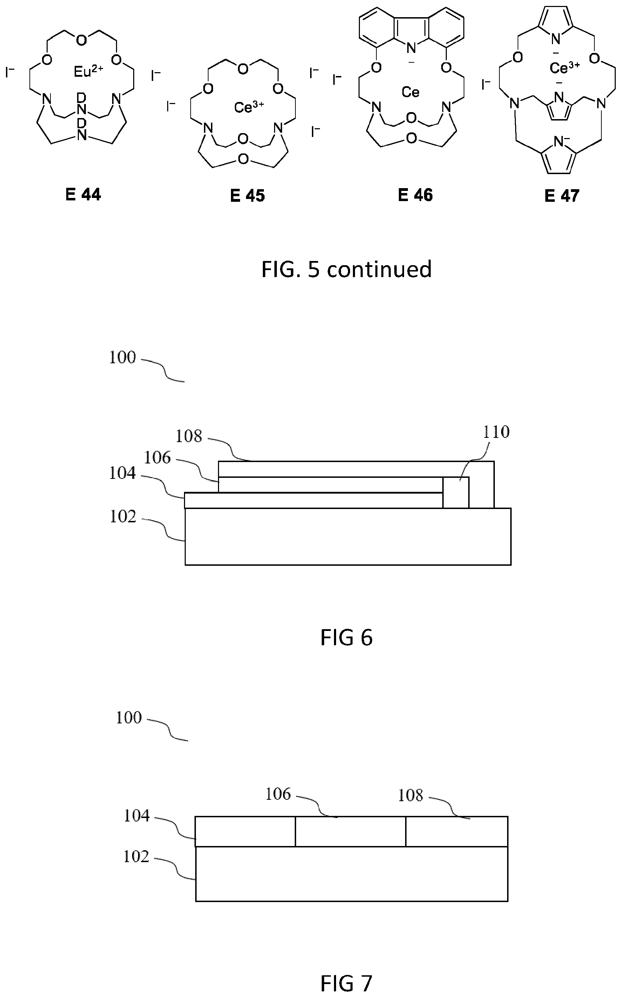

In preferred embodiments of the coordination compounds of the present invention, the sum of the negative charges of the covalently attached anionic groups matches the charge of the central lanthanide cation and thus the ligand,

by coordinating the central lanthanide cation, forms a complex of neutral charge. In such configuration no unbound negatively charged anions are present, therefore anion drift in device configuration under applied electric fields is elegantly circumvented. For example, if the central lanthanide is chosen to be divalent Europium, Eu2+, then the ligand may contain exactly two singly negatively charged anionic groups or exactly one twofold negatively charged anionic group. Thus, ligand examples L17-L19, reference L37, L57-L64, and L101 are preferably chosen to complex a divalent lanthanide, such as Eu(II). Examples of complete coordination compounds according to the invention E14 - E16, and E30 shown in **FIG. 5** additionally serve for illustration for the case of two singly negatively charged anionic groups combined with divalent Europium as central cation. Three singly negatively charged anionic groups are present for example in reference ligand L95 with three pyrroles in Fig 1. Such ligands are especially suited in cases not according to the invention where the central lanthanide is in oxidation state +3, for example for Ce3+ or Er3+. Alternatively, such a charge neutral metal organic Ln(III) reference compound may contain one singly combined with one twofold negatively charged anionic groups or alternatively only one threefold negatively charged anionic group is for example covalently attached at positions **R** to the ligand according to formula (1-1) or (1-2). Within the scope of the invention are situations where there is a singly or negatively charged ligand and one non-covalentl anion present in the coordination compound, this is exemplified in reference E17, where a Ce(III) is complexed by a singly negatively charge polycyclic ligand according to formula (1-1) with two more iodides being part of the complex resulting in an overall charge neutral reference coordination compound.

The negatively charged anionic side groups depicted in **Fig. 3** shall not limit the scope of the invention. In various embodiments, anionic groups covalently linked to the organic polycyclic ligand may be twofold or even higher charged, for example by attaching sulfate, phosphate, or chromate anionic groups. Similarly, the polycyclic organic ligand may be negatively charged higher than 3. In such a case, a charge neutral compound may be obtained by incorporation of additional suitable organic or inorganic cations, such as ammonium, alkali, alkaline earth metal cations or similar into the coordination compound according to invention. Whilst any of the anionic side groups depicted in **Fig. 3** may be used, preference is generally given to chemically soft side groups that do not strongly interact with the optically excited central cation. As such preference is given to anionic side groups that do not tend to form direct chemical bond with the central cation, but where instead the negative charge is well shielded and distributed over many atoms. Additionally, preference is given to anions that do not substantially absorb in the visible spectral region, especially not in the deep blue spectra region which is between 430-500 nm. Very well suited in this context and therefore preferred are substituted or non-substituted closo-carboran cluster anions such as the generic structures b26 and b27 in Fig 3 or, for clarity redrawn here as (1-25) and (1-26):

C
BRb
−
(1-25)
(1-26)

with $\mathbf{R_b}$ independently in each occurrence representing hydrogen, deuterium, halogen - preferably chlorine -, -, or straight or branched alkyl chain radicals including cycloalkyl radicals, the alkyl groups containing from one to fifteen, preferably one to ten, more preferably one to five carbon atoms, the term "cycloalkyl radical" referring to monocyclic, polycyclic, and spiro alkyl radicals containing 3 to 12, preferably 3 to 8 ring carbon atoms, preferably $C_{1-3}$-alkyl, more preferably methyl. The dashed line indicating the covalent bond to the remaining polycyclic ligand according to formulae (1-1) or (1-2) for example at positions R. Examples of ligands according to formula (1-1), with two of the preferred anionic side groups according to formula (1-25) being covalently attached, thus providing a twofold negatively charged ligand that is ideally suited to complex a divalent lanthanide such as Eu(II), are illustrated as L17-L19 in Fig. 1.

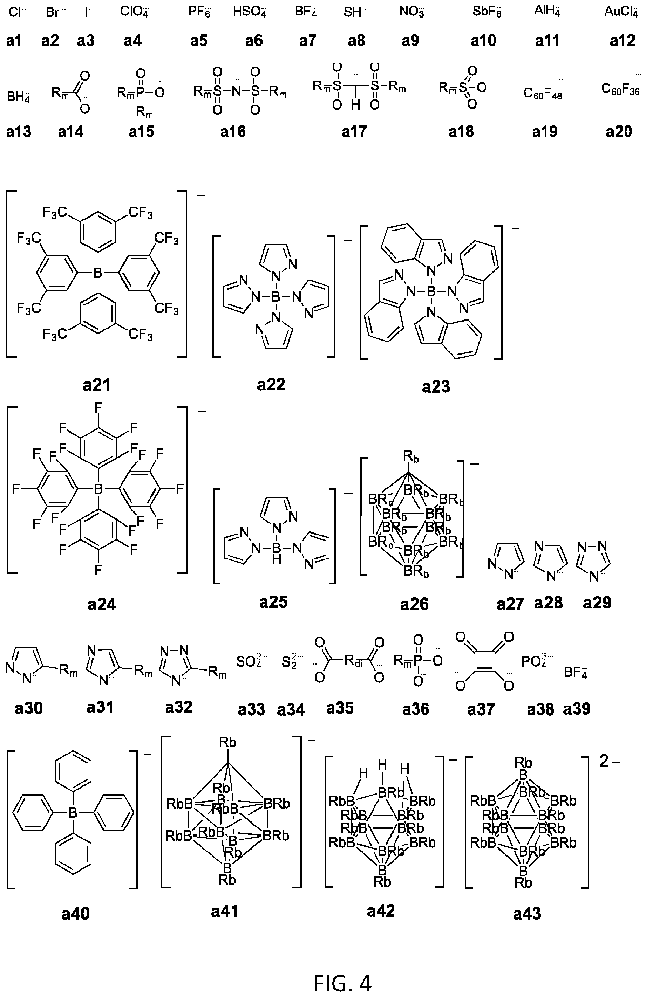

In various embodiments, the coordination compound may include at least one negatively charged anion that is not covalently bound to the polycyclic organic ligand according to formulae (1-1) or (1-2). For illustration some suitable anions are illustrated in a1-a43 in **Fig. 4,** wherein $R_b$, $R_m$, $R_{di}$ have the same meaning as for **FIG. 3** above. The negatively charged anion may be a small inorganic anion such as, but not limited to, a1 to a13 in **FIG. 4.** In Fig. 5 examples **E18-E26,** and reference **E17**,coordination compounds according to formula (1-1) comprising divalent Europium and small anions selected from a1 to a13, are depicted for illustration purpose. When an external field is applied to a layer comprising the coordination compound according to invention, for example in operative OLED device configuration, anions may drift

towards oppositely charged electrodes, especially when using small and non-bulky anions. Such behaviour may define a so-called light emitting chemical cell.

A light emitting chemical cell may be an embodiment of an OLED in this description. Drift of charged species within a device including the lanthanide coordination compound having an aza-cryptand ligand according to formulae (1-1) or (1-2) may lead to very low driving voltages, which may assist to facilitate very good power efficiencies. This may be desirable for some applications, such as general illumination or signage. Yet, for other applications that require fast response times, for example flat panel displays, such ion drift may lead to time dependent current-voltage-luminance (IVL) characteristics, which may be difficult to control, and may not be desirable as such. Therefore, the choice of the anions may depend strongly on the application.

In various embodiments, the coordination compound may contain comparably large and bulky organic anions. Such anions may be employed if ion drift is not desired. Examples of such anions include fluorinated or non-fluorinated fullerene (C60-, C60F36-, C60F48-) fluorinated aryl, closo-boranes, *closo*-carboranes, and borates, Examples a14 to a26 and a40 to a43 thereof are illustrated in **FIG. 4** but are not limited thereto. E2 to E8 and E27 to E29 as well as E38 to E41 depicted in **Fig. 5** illustrate the use of two identical bulky anions in combination with divalent Europium coordinated by polycyclic ligands according to formula (1-1).

The negatively charged anion may include more than one atom, preferably more than three atoms, and/or can have a molecular weight of at least 128 g/mol. Such larger anions are generally more bulky or heavier and as such are better suited for applications where drift of anions is not desired.

In various embodiments, anions without substantial absorption in the visible spectral region, and in particular in the deep blue spectral region between 430-500 nm, may be used. In case there are two or three non-covalently bound anions, they may be the same or of different type.

In various embodiments, anions comprising bor, known as borates, or boron clusters may be used.

In various embodiments, anions with individual negative charge of -2, or -3, or -4 are present in the coordination compound according to the invention, examples a33 to a38 and a43 thereof are illustrated in **FIG. 4.**

As with the embodiments using covalently attached anionic groups, preference is given to anions that do not strongly interact with the optically excited central cation. As such, preference is given to anions that do not tend to form direct chemical bonds with the non-excited or excited central cation, but where instead the negative charge is well shielded and distributed over many atoms. Very well suited in this context, and therefore preferred are substituted or non-substituted boranes of the *closo-* and/or *nido-type* or closo-carborane cluster anions such as a41 to a43 or a26 in Fig.4 or partly redrawn for clarity below as (1-22) - (1-24):

(1-22) (1-23) (1-24)

with $\mathbf{R_b}$ having the same meaning as in Fig 3 and 4. Those particular anions as well show the desired property of high triplet energy (>2.7eV) combined with low absorption in the visible spectral region. In Fig. 5 examples using those preferred anions in combination with divalent Europium complexed by a ligand according to formula (1-1) are E2 to E8.

The at least one lanthanide coordinated by the ligand according to formulae (1-1) or (1-2) is Eu2+. In other words, Europium in oxidation state +2 is coordinated by an organic ligand represented by the formulae (1-1) - (1-21). With Eu2+, the small rigid cavity of the ligand according to formulae (1-1) and (1-2) with only 10-14 atoms in the aza crown basis ring provides a size discriminating ligand that stabilizes the divalent over the trivalent oxidation stage of the central cation. In this way, oxidation of the atypical divalent into the common trivalent oxidation state is prevented for the lanthanide. Thus, a metal organic coordination compound is provided that is highly stabilized against oxidation such that processing at ambient conditions becomes possible.

Eu2+ has an open shell configurationwith color pure single peak emission spectrum for Eu2+. Examples of coordination compounds based on Eu2+ according to the invention are shown in **FIG. 5** as E1-E10, E12-E16, E18-E43, respectively.

In various embodiments, the coordination compound according to the invention may be used in a mixture with at least one second electrically neutral organic compound, whereby the coordination compound according to the invention may be present in a volume ratio between 0.5 and 99.5 vol%, based on the mixture which is 100 vol%.

In various embodiments, the second organic material (b) may serve as charge transporting host to enable electrical excitation of the coordination compound (a) present in the mixture according to the invention. To achieve this desired functionality without severely compromising the triplet energy level, the second charge-neutral compound in preferred embodiments comprises (hetero)arene fragments selected from benzene, pyridine, pyrrole, furan, carbazole, dibenzofuran, triazine, triazole, or benzofuran with one or more hydrogen atoms removed.

In various alternative embodiments the second charge-neutral compound (b) has a molecular weight $M_n$ above 1000 g/mol, and the coordination compound (a) and the second charge-neutral compound (b) are chemically linked with each other, for example by a covalent or coordinative chemical bond. Here the mixture is a polymeric compound, which might have benefits in terms of very high glass transition temperatures, or it may ease the processing of the mixtures using wet solution techniques.

In various preferred embodiments, the mixture according to the invention is used in an organic electronic device, comprising a first electrode, a second electrode, and a first organic layer arranged such that it is electrically interposed between the first and second electrodes, wherein the organic layer comprises a mixture according to the various embodiments described above, preferably a composition containing at least 50 wt-%, more preferably at least 80 wt-%, most preferably at least 95 wt-% of the mixture of (a) and (b) as emitter and host materials. Preferably no host apart from compound (b) is employed in the (first) organic layer. Further preferred are embodiments, with the organic electronic device being an organic light emitting diode, emitting preferably substantially in the blue spectra region at < 500 nm. The term "substantially" is defined as above and generally implies that the wavelength peak of the emission spectrum is at < 500 nm. In other words, the electroluminescent device emits mostly in the desired deep blue spectral region urgently needed for consumer display applications.

In various embodiments, the coordination compound (a) according to various embodiments may be dispersed into a matrix (b) having suitable optical properties, such as, for example, a high transparency in certain desired parts of the visible spectrum. If this matrix is brought in optical contact to a light source emitting at a sufficiently short wavelength, this light may be absorbed by the coordination compound and reemitted at a substantially longer wavelength. A suitable light source may for example be a light emitting diode, emitting light at a wavelength (substantially) shorter than 430 nm, which may be reemitted by the coordination compound at a wavelength longer than 430 nm. The matrix may have any physical dimensions. It may for example be a thin layer of 10 nm to 10,000 nm. The matrix may as well be of granular form or in form of small particles of average diameter between 10 nm and 100,000 nm. For use in optical devices, in the latter cases, the matrix may be applied inside another host material for support.

The combination of unique emission properties with the ease of processing makes the coordination compounds according to various embodiments highly attractive for application in organic electronic devices. In this description an organic electronic device may be any device or structure including substantially organic layers or subunits, where an electro-magnetic stimulus may be converted into an electrical field or current or vice versa, or where an input electrical current or electrical field may be used to modulate an output electrical current or electrical field.

In one embodiment, the organic electronic device including the coordination compound according to various embodiments may be used as semiconducting organic material in an organic field-effect transistor or an organic thin-film transistor.

**FIG. 6** and **FIG. 7** illustrate embodiments of an organic electronic device 100 configured as optically active device. The organic electronic device may include a first electrode 104, e.g. on a substrate 102 or as the substrate; a second electrode 108; and an organic layer 106 arranged such that it is electrically interposed between the first and second electrodes 104, 108. The first and second electrodes 104, 108 may be electrically insulated from each other by an insulating structure 110, e.g. a resin or polyimide. The first and second electrodes 104, 108 may be stacked over each other (FIG. 6) or may be arranged in a common plane (FIG. 7).

The organic layer 106 may include the coordination compound according to any of the described or illustrated embodiments, e.g. as a pure compound, or in a mixture as described before.

In various embodiments, the organic electronic device 100 may be an optoelectronic device, the optoelectronic device being at least one of an organic light emitting diode (OLED), a light emitting cell (LEC), an organic photodetector, or a photovoltaic cell. That is, photons from an external electromagnetic field may be absorbed in the organic layer 106 and converted into current by means of an electrical field between the first and second electrodes 104, 108. Such a device would be a photodiode (oPD) and its primarily use may be to sense external light. It would be an organic photovoltaic (OPV) device, if the primarily use is to convert light into current.

The organic layer 106 is arranged electrically between the first and second electrodes 104, 108 such that an electronic current may flow from the first electrode 104 through the organic layer 106 to the second electrode 108 and vice versa during operation, e.g. in light emission applications. Alternatively, in photoelectric applications, a charge carrier pair may be formed in the organic layer 106 and charge carriers of the charge carrier pair may be transported to the first and

second electrodes 104, 108 respectively. In other words, in light emission applications, upon application of sufficient voltage, holes and electrons are injected from the anode and the cathode, respectively, and drift towards the organic layer 106, where charges of opposite sign recombine to form a short-lived localized excited state. The short-lived excited state may relax to the ground state thereby giving rise to light emission.

The first and second electrodes 104, 108 may be substantially unstructured layers, e.g. for general lighting applications, or may be structured, e.g. for light emitting diodes or transistors for pixels in a display application.

The organic electronic device 100 may be configured to emit substantially monochromatic light such as red, green, blue, or polychromatic light such as white. The light may be emitted through the first electrode 104 (bottom emitter), through the second electrode 108 (top emitter), or through first and second electrodes 104, 108 (bidirectional emitter). The light may as well substantially be emitted in a direction parallel to the organic layer 106 using suitable opaque electrodes 104, 108. In such a layout lasing may be achieved, and the device may be an organic laser, which, in this description, may be considered as a specific type of electroluminescent devices. The coordination compounds according to various embodiments may have excellent emission properties, including a narrow, deep blue emission spectrum with short excited state lifetime. Given their high atomic weight and doublett and septett multiplicities, the optical transitions in particular of $Ce^{3+}$, and $Eu^{2+}$ may be as well widely indifferent to excitation with either spin 1 or spin 0 electron-hole pairs. In other words, they harvest singlet and triplet excitations, giving high light emission efficiency. As such, the coordination compounds according to various embodiments may be ideally suited for application in organic electroluminescent devices, such as organic light emitting diodes (OLED). In this description, an electroluminescent device may be any device including an organic layer disposed between and electrically connected to an anode 104/108 and a cathode 108/104. Upon application of sufficient voltage, holes and electrons may be injected from the anode 104/108 and the cathode 108/104, respectively, and drift towards the organic layer 106, where charges of opposite sign recombine to form a short-lived localized excited state. The short-lived excited state may relax to the ground state thereby giving rise to light emission. Relaxation pathways without light emission, such as thermal relaxation, may be possible too, but may be considered undesirable, as they lower the conversion efficiency of current into light of the device. Organic light emitting cells (LECs) may be considered as a subclass of OLED devices that comprise of mobile charged species inside the active organic layer 106, which are able to drift inside a external applied electrical field. As such OLEDs encompass LEC type devices.

Further layers may be formed and in electrical connection between the first and second electrodes 104, 108, e.g. configured for charge carrier (electron or hole) injection, configured for charge carrier transport, configured for charge carrier blockage or configured for charge generation. The recombination of electrons and holes may as well happen at the interface of the organic layer 106, which contains the coordination compound according to the invention and an adjacent layer, such an electron blocking layer. Further optically functional layers, e.g. a further electroluminescent material and/or a wavelength conversion material may be formed electrically between the first and second electrodes 104, 108 and in the optical path of the organic layer 106, e.g. on top of the second electrode 108 and/or on the opposite side of the substrate 102. In addition, encapsulation structures may be formed encapsulating the electrically active area, e.g. the area in which an electrical current flows, and may be configured to reduce or avoid an intrusion of oxygen and/or water into the electrically active area. Further optically functional layers, e.g. an antireflection coating, a waveguide structure and/or an optical decoupling layer may be formed within the optical light path of the organic layer 106.

As example, hole or electron blocking layers may be used to optimize the individual hole and electron currents through the organic electronic device 100. This may be known to those skilled in the art as charge balance in order to optimize efficiency and operational stability. In various embodiments, dedicated hole or electron charge transport layers may be present in the organic electronic device 100 to space the emission region from the first and second electrodes 104, 108.

Examples of hole transport materials include known materials such as fluorene and derivatives thereof, aromatic amine and derivatives thereof, carbazole derivatives, dibenzofuran, dibenzothiophene, and polyparaphenylene derivatives. Examples of electron transport materials include oxadiazole derivatives, triazine derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and its derivative, diphenoquinone derivatives, and metal complexes of 8-hydroxyquinoline and its derivatives and various derivatives of silanes.

In various embodiments, those charge transport layers may include electrical dopant molecules or metals or may be in contact to charge injection layers. Any of those auxiliary layers may be fully organic or may include inorganic functional moieties. For example, charge transport layers may be made of the class of Perovskite materials.

The coordination compound or mixture as illustrated or described in any one of the embodiments may be used as a pure organic emitting layer 106 of any thickness in the range of 0.1 nm and 100 nm, preferably in the range of 0.1 and 10 nm.

The organic layer 106 may in various embodiments include charge transport materials to improve charge transport into and through the organic layer 106. Charge transport materials may be any material that is able to transport

either holes or electrons or both types of charges. In particular a charge transport material may be any aryl, or heteroaryl organic compound or any metal complex or any mixture thereof. Examples of materials present in the light emitting layer 106 include carbazole derivatives, including carbazole-phosphine oxide materials, oxadiazole derivatives, triazine derivatives, silane derivatives and any other material or combination of materials with sufficiently high triplet level that may transport charges or otherwise benefit the device performance. The volume percentage of the coordination compound as a function of the combined charge transport materials may be between 0.5 and 99.5 vol% in the organic layer 106.

In various embodiments, the coordination compound of the organic layer 106 may have a higher hole affinity compared to all charge transport materials present in the organic layer 106. This is deemed necessary to ensure hole trapping during operation as an electroluminescent device. Thus, hole capture/trapping of for example Ce(III) $\rightarrow$ Ce(IV) or Eu(II) $\rightarrow$ Eu(III) is considered a necessary first step to excite the central optically active metal in the metal organic coordination compound according to the invention. Additionally, **the positively charged** metal organic complex according to the invention may have a higher electron affinity compared to all charge transport or other materials present in the organic layer 106 to ensure electron capture and thereby excitation of the metal organic complex. Thus, electron capture by the 5d orbital of the positively charged metal organic complex for example Ce(IV) $\rightarrow$ Ce(III)* or Eu(III) $\rightarrow$ Eu(II)* is considered a necessary second step to excite the central optically active metal in the metal organic coordination compound according to the invention. Both these relative energy level requirements are of particular advantage if the metal organic complex according to the invention shall be electrically excited and is meant to emit photons, in particular if the desired application is an electroluminescent device, i.e. an OLED. For other applications, where light generation is not the primarily application of the complex, such as photodiodes or photodetectors, other requirements for the surrounding host matrix may apply.

A wide variety of techniques on how to measure electron and hole affinities for organic materials have been published in the literature. However, for the purpose of various embodiments, the particular technique of how to measure the oxidation and or reduction potential is not essential, e.g. only the relative order between the coordination compound and the charge transport materials may be of importance. Oxidation/reduction potentials may for example be deducted using quantum mechanical computing techniques based on density functional theory and experimental techniques are very well known in the art, e.g. see R.J.Cox, Photographic Sensitivity, Academic Press, 1973, Chapter 15.

The organic layer 106 may contain any further organic or inorganic material in a range of 0.1 to 99.9 vol% based on the layer 106 that is 100 vol%, that are not intended to transport charges. For example, the organic layer 106 may include polymers (in a mixture or as a compound) which may be added to improve film quality and prevent crystallization. Other materials may be added to evenly space the coordination compound inside the organic layer 106.

In color conversion materials, the coordination compounds according to various embodiments may allow the ease of processing using vacuum based techniques. In other words, the evaporation temperature of the compounds including the lanthanides cation may be sufficiently low to allow for thermal vacuum processing techniques to be used. Reduced evaporation temperatures can be achieved according to the invention by using the metal-organic coordination compounds as here the ionic character of the compounds is strongly suppressed, if compared to similar organic salts without covalently attached monoanionic groups. Consequently, the evaporation temperature is reduced and incorporation into organic electronic devices becomes possible using state-of-the-art vacuum deposition techniques.

In a preferred embodiment, the mixtures according to the invention emit substantially in the deep blue spectral region below 496 nm, preferably below 477 nm, or even more preferred below 459 nm. By example of a single Gaussian emission spectrum, as often observed for Eu(II) coordination compounds, this implies that the peak of the emission spectrum is preferably below 496 nm, preferably below 477 nm, or even more preferred below 459 nm. To confine the excitation energy on a such deep blue emitting coordination compound, in a preferred embodiment, the second organic host compound (b) has a triplet energy preferably higher than 2.5 eV, more preferably higher than 2.6 eV, and most preferably higher than 2.7 eV. In this context, "substantially" means that at least 50%, preferably at least 65%, more preferably at least 80% of the emission or of the integral under the emission curve are below the above thresholds.

The excitation of the light emitting coordination compound may be energetically confined. This way, high efficiencies may be achieved. Thus, confinement layers may be formed adjacent to the organic layer 106, wherein the confinement layers may have a triplet energy $T_1$ higher than 1.8 eV, e.g. higher than 2.3 eV, e.g. higher than 2.7 eV. Similarly, any material of the organic layer 106 (other than the electroluminescent compound) may have a triplet energy $T_1$ higher than 1.8 eV, e.g. higher than 2.3 eV, e.g. higher than 2.7 eV.

In various embodiments, the organic electronic device 100 includes two or more subunits each including at least one light emitting layer. The subunits may be stacked over each other physically separated and electrically connected by a charge generation layer or, alternatively, may be arranged side by side. The subunits may be subpixels of a pixel in a display or general lighting application. The light emitted by the subunits may be mixed to generate a light of a predetermined color. Each subunit may emit light of the same or a different color. The overall light emitted by such organic electronic device 100 may contain a narrow spectral region, such as blue, or may contain a wide spectral region such as white, or a combination thereof. The coordination compound illustrated or described in any one of the

embodiments may or may not be present in all subunits of the organic electronic device 100.

In various embodiments, the light emitted by the organic electronic device 100 may be in optical contact to at least one optically active layer, including any optically active materials such as organic molecules or quantum dots. The optically active layer may be a spectral filter element, which may absorb part of the light emitted by the organic electronic device 100. In another embodiment, the optically active layer may absorb at least part of the light emitted by the organic electronic device 100 and may reemit it at longer wavelength (wavelength conversion), e.g. by quantum dots.

As example, the organic layer 106 may be configured to emit light substantially at wavelengths shorter than 500 nm and the optically active layer may be configured to substantially reemit light at wavelengths longer than 500 nm. The optically active layer may be placed in between the anode and cathode of the organic electronic device 100 or outside of it. The optically active layer may as well be part of the organic layer 106.

The organic electronic device 100 may be configured as a large area OLED device used for illumination, signage, or as a backlight. Alternatively, the organic electronic device 100 may include a plurality of OLEDs arranged in a pixilated layout (plurality of OLED pixels) that are individually connected electrically, e.g. for flat panel display applications. Here, individual pixels may have the capability of emitting light of substantially narrow spectral portions; especially of red, green, and blue. The coordination compound may or may not be present in any of the individual pixels.

In another embodiment, the individual pixels may be configured to emit white light. Red, green, and blue spectral portions are generated by using suitable filter elements in optical contact with the respective pixelated OLEDs.

In another embodiment, the OLED pixels emit blue light and the red and green spectral portions may be generated by using a suitable color conversion element in optical contact with the OLED pixels.

In another embodiment, the OLED pixels emit substantially in the near-UV spectral region with wavelength $<450$ nm and the red and green and blue spectral portions may be generated by using a suitable color conversion element in optical contact with the OLED pixels.

In various embodiments, the organic electronic device 100 includes an anode 104; a cathode 108; and an organic layer 106 disposed between the anode 104 and the cathode 108. The organic layer 106 includes an electroluminescent coordination compound according to various embodiments. The coordination compound includes at least one lanthanide coordinated by a cyclic organic ligand according to the present invention according to formulae (1-1) or (1-2).

The coordination compound may be imbedded into at least one second electrically neutral organic compound. The second organic compound may have a triplet energy higher than 2.5 eV, more preferably higher than 2.6 eV and even more preferably higher than 2.7 eV. The coordination compound may have a higher hole affinity compared to the second organic compound.

An organic electronic device 100 according to various embodiments may be fabricated using a wide range of commonly used techniques, including, but not limited to, deposition of all or some layer, from gas phase vacuum deposition, solution phase, or gas phase using a carrier gas method.

Due to the asymmetry of the macrocyclic ligands according to formulae (1-1) and (1-2) many metal organic complexes according to the invention show a relatively low dipole moment allowing for thermal deposition from gas phase with high yields. Thus, in various embodiments, deposition via the gas phase in vacuum may be used, whereby the coordination compound may either undergo sublimation or evaporation. The coordination compounds according to the invention show an improved sublimation/evaporation yield. The transfer into the gas phase may be improved by using a carrier gas technology, whereby an inert gas that may not be deposited into the organic layer may be helping the sublimation or evaporation of the coordination compound to be deposited. During the deposition process from gas phase, the coordination compound may as well be co-deposited with one or more material to fabricate any desired mixed layers.

Another preferred technique to fabricate layers including the coordination compound according to various embodiments may be deposition from a liquid phase using a mixture or a single organic solvent, whereby the coordination compound according to various embodiments may be dissolved or forms a suspension within the organic solvent; in this description may be referred to as the ink. The ink using this deposition process, may include a wide variety of other materials apart from the coordination compound according to various embodiments to allow fabrication of mixed layers from solution. Additives within the ink may for example, but may not be limited to, be organic or inorganic materials capable of transporting charges, materials that improve the film formation, materials that improve the distribution of the coordination compound within a host material, organic or inorganic materials that improve the efficiency of the device, e.g. by reducing the refractive index. The deposition from solution may not be limited to any specific technique. Examples of the deposition from solution include spin coating, casting, dip coating, gravure coating, bar coating, roll coating, spray coating, screen printing, flexographic printing, offset printing, inkjet printing. The improved solubility of the coordination compounds according to the present invention in non-polar solvents eases the deposition from solution and makes it advantageous. In particular, solvents with relatively low polarity index such as tetrahydrofuran or toluene may be used as part of the ink formulation.

Various post processing techniques may be applied to improve the performance or stability of the organic electronic device. In one embodiment, some or all layers of the organic electronic device include functional groups capable of chemically crosslinking upon thermal or optical exposure thereby forming larger covalently bound molecules with

improved physical properties.

In various embodiments, the coordination compound may be formed in-situ using deposition from solution. Here, the organic ligand according to formulae (1-1) or (1-2) of the present invention, excluding the lanthanide metal, may first be deposited onto a suitable substrate or other organic layer thereby forming a seed layer. Any suitable technique may be used to fabricate this seed layer. This seed layer including the organic ligand may include any other material. Preferred may be organic charge transport materials, for example suitable to achieve hole transport for organic electronic devices. Preferred may be further additional inert organic or inorganic materials that aid the layer formation or improve its thermal stability or improve the distribution of the organic ligand within this seed layer. In a next deposition step and sequential to forming the seed layer including the organic ligand according to the invention excluding the metal, a layer including a molecular salt including comprising the lanthanide may be fabricated using a solution process. The lanthanide compound may be any charge neutral compound including a lanthanide in an oxidation state 2+, 3+, 4+ and any number of suitable anions. The anions may as well be covalently bound to a suitable polymer. The ink including the lanthanide compound may as well include one or more additive to fabricate mixed layers. These additives may be organic or inorganic materials to aid charge transport, may be organic or inorganic materials that improve the efficiency of the device, or may be any material that improves the film formation. At the interface of the seed layer including the organic ligand according to the invention, the solubilized lanthanide metal compound will interact with the macrocyclic organic ligands according to formulae (1-1) or (1-2) to form the coordination compound according to various embodiments in-situ. Alternatively, the process of first forming the layer containing the ligand and secondly processing the lanthanide compound may be inverted. Preferably, the metal-organic coordination compound is used directly.

For one or more aspects, at least one of the components set forth in one or more of the preceding figures may be configured to perform one or more operations, techniques, processes, and/or methods as set forth in the example section below.

## EXAMPLES

The examples set forth herein are illustrative and not exhaustive.

Any of the examples may be combined with any other example (or combination of examples), or coordination compounds described therein may be combined with the above embodiments of the invention, unless explicitly stated otherwise. The foregoing description of one or more implementations provides illustration and description, but is not intended to be exhaustive or to limit the scope of aspects to the precise form which is defined by the claims.

Modifications and variations are possible in light of the above teachings or may be acquired from practice of various aspects. Any of the above-described embodiments may be combined with any other example (or combination of examples), unless explicitly stated otherwise.

### Examples Calculated with Quantum-Chemical Methods

*Computational Protocols*

The computational protocol to determine structures, dipole moments (DM), emission energies (EE), and the ionization potentials (IPs) of the respective Eu(II) complexes their electronic ground ($4f^7 5d^0$) and first (energetically lowest) excited ($4f^6 5d^1$) state consists of 3 main steps, all of which can be accomplished with freely available open-source software (xTB: https://github.com/grimme-lab/xtb;
CREST https://github.com/crest-lab/crest; CENSO https://github.com/grimme-lab/CENSO) and the commercially available quantum-chemical program package ORCA (https://www.faccts.de/orca/).

These three steps are firstly, the generation of a large comprehensive ensemble of all possible structures of given complex with fast semi-empirical methods, secondly a multi-level energetical screening, optimization, and reranking of the ensemble with increasingly accurate density-functional theory (DFT) to determine the relevant (energetically lowest) conformer(s), and thirdly the calculation of the desired properties with a DFT methodology.

In principle, any quantum-chemistry software can be used for these calculations and would provide near-identical results if the methods described in detail below (functionals, dispersion corrections, basis sets, geometric Counter Poise corrections, effective core potentials, solvent models and parameters) are applied in the same way.

Due to the statistical (non-deterministic) nature of step 1 in this approach, the results are not exactly reproducible. However, if the simulations in step one are sufficiently long, the approach will always yield the same relevant (lowest energy) conformer after step 2, which will have very similar properties in the calculations in step 3.

As a very conservative estimate, the precision of the predicted dipole moments (over repeated runs for the same molecule) should be $\pm 1$ D and $\pm 0.1$ eV for emission energies and ionization potentials.

The three main steps of the computation approach are:

1. **Generation of a comprehensive conformer ensemble** with the CREST program version 2.12 using the implemented semi-empirical tight-binding model GFN2-xTB and the Analytical Linearized Poission-Boltzmann (ALPB) solvation model with parameters for toluene as implemented in the xTB software version 6.4.1.

The conformer generation uses at least two iterations with seven parallel metadynamics simulations, each with at least 30 picoseconds (ps) of simulation time (420 ps total), but more can be necessary for specific complexes. Since CREST by default excludes all molecules with topological changes in the bonding pattern compared to the starting structure (as these are isomers not conformers), changes on the central Eu-atom are have to be explicitly allowed by excluding Eu from the topology check (--notopo Eu). This is required to ensure that structures with different coordination geometries and numbers of ligand-atoms attached to Eu are also considered (e.g. an iodine coordinated to Eu(II) can move away and coordinate to an NH-proton instead). If isolated (not covalently bound) anions are included in the complex like iodide or closoborane, the NCI mode of CREST is invoked (-nci -wscal 1.5). All structures within 8 kcal/mol of the lowest structure are kept in the ensemble (and are thus included in step 2).
Commandline prompt for the input-structure input.xyz:
*crest input.xyz -gfn2 -alpb toluene (-nci -wscal 1.5) -mdlen 30 -nmtd 7 -notopo Eu -ewin 8*

2. **Multi-level screening, optimization, and reranking of the conformer ensemble** from 1. in 4 sub-steps with DFT methods of increasing accuracy using the CENSO program as driver and ORCA 5.0.3 to carry out the DFT calculations.

**(A) Prescreening** based on PBE-D4/def2-SV(P)+gCP, single-point energies using the structures from step 1 (notation PBE-D4/def2-SV(P)+gCP//GFN2/ALPB(toluene)). Solvent effects are included by adding the GFN2-xTB/ALPB free energy of solvation (CENSO default) and the 28-electron def2-ECP effective core potential is used for Eu and all atoms heavier than krypton (ORCA default). All conformers whose energy is within 7 kcal/mol of the lowest conformer at this level of theory are included in step 2B. *Commandline prompt: censo -inp crest_conformers.xyz -solvent toluene -u 7 - chrg 0 -prog orca -part0 on -func0 pbe-d4 -part0_threshold 7*
**(B) Screening** based on r2SCAN-3c, single-point energies calculated for the structures from step 1 using the SMD model with parameters for toluene to include solvent effects ($r^2$SCAN-3c/SMD(toluene)//GFN2-xTB/ALPB(toluene)). The resulting single-point energies are combined with thermostatistical contributions (zero-point vibrational energy, entropic and enthalpic vibrational, translational and rotational contributions) evaluated at the GFN2-xTB/ALPB(toluene) level of theory using the modified rigid-rotor harmonic-oscillator (mRRHO) approximation (CENSO default) to obtain free energies. All conformers with a free energy that is within 4.5 kcal/mol of the lowest conformer at this level of theory are included in step 2C.
*Commandline prompt: censo -inp crest_conformers.xyz -solvent toluene -u 7 - chrg 0 -prog orca -part0 on -func0 pbe-d4 -part0_threshold 7 -part1 on - part1_threshold 4.5*
**(C) Reoptimization** of the structures (from step 1) with $r^2$SCAN-3c using the SMD solvent model with parameters for toluene and the "lax" convergence criteria setting of CENSO. Entropic and enthalpic contributions are included at the GFN2-xTB/ALPB(toluene) theory level using mRRHO approximation combined with the single-point Hessian approach (CENSO default). All conformers with a free energy that is within 1.5 kcal/mol of the lowest conformer at this level of theory are included in step 2D.
*Commandline prompt: censo -inp crest_conformers.xyz -solvent toluene -u 7 - chrg 0 -prog orca -part0 on -func0 pbe-d4 -part0_threshold 7 -part1 on - part1_threshold 4.5 -part2 on -optlevel2 lax -part2_threshold 1.5*
**(D) Final Reranking** of the structures from 2C using singlet-point energies calculated with the wB97X-V functional using the def2-TZVPP basis set and def2-ECPs and the SMD(toluene) model for solvent effects. Thermostatistical corrections (enthalpy, entropy) are taken from step 2C.
If there is more than one structure within 1.0 kcal/mol of the lowest structure at this level of theory and the respective structures are similar, then only the lowest structure is taken to the next step (3). If there are two or more structures within 1 kcal/mol with different coordination geometries (different atoms coordinating to Eu, different coordination geometry), they are all included in the next step.
*Command-line prompt: censo -inp crest_conformers.xyz -solvent toluene -u 7 -chrg 0 -prog orca -part0 on -func0 pbe-d4 -part1 on -part2 on -part3 on -func3 wb97xv -basis3 def2-tzvpp*

3. **Calculating of DM, EE and the IPs for the lowest-lying conformer(s)** from 2D with the wB97X-D3 dispersion-corrected density functional (ORCA keyword !wB97X-D3) using the def2-TZVP basis set and def2-ECPs 28-electron pseudopotential for Eu, the def2-SVP basis set (and if necessary def2-ECPs) for all other atoms, the geometrical Counter Poise correction (ORCA: gCP(DFT/SVP)) for basis set incompleteness error, and the SMD solvation model with (slightly modified, see below) parameters for anisole. Convergence thresholds for geometry optimizations in

ORCA are set to "loose" (!LOOSEOPT).

- **DM: Dipole moments** are determined by optimizing the geometry of the lowest conformer from 2D in its electronic ground state at the wB97X-D3 level of theory as specified in 3, and calculating the electric dipole moment for the optimized structure.
- **EE: Emission energies** are calculated by optimizing the lowest electronically excited state of the lowest conformer from 2D with time-dependent (TD-)DFT/wB97X-D3 in the Tamm-Dancoff approximation (TDA) using the basis sets, corrections as specified in 3. For the excited-state optimization, the electronic part of the SMD solvation model (C-PCM) is replaced with the linear-response polarizable-continuum model (LR-PCM) using equilibrium solvation for the gradient calculation during the optimization, and non-equilibrium solvation for final emission energies (ORCA default). The emission energy corresponds to the energy of the first excited state after the optimization is converged.
- **IP of Eu(II) in the ground state** is calculated as the difference between the electronic energy of the fully relaxed complex with ground-state Eu(II) and the energy of the fully relaxed complex with Eu(III) (single positive charge) according to the following equation: IP = E(Eu2) - E(Eu3). The optimizations use the wB97X-D3 level of theory as specified in 3.
- **(D) IP of Eu(II) in the lowest excited state** is calculated as the electronic energy difference between the geometry-optimized complex with Eu(II) in its first excited state and the corresponding geometry-optimized complex with Eu(III) (Eu3, single positive charge) according to the following equation:

$$IP = E(Eu2^*) - E(Eu3).$$

Optimization of the electronically excited Eu(II) complex uses the same TDA-wB97X-D3 level of theory specified in 3B. Optimization of the Eu(III) complex uses the same ωB97X-D3 level of theory specified in 3 (sample inputs S2 and S3).

**Technical Details and Sample Inputs:**

The workflow described in step 1. and 2. of this protocol is taken from the following article with some modifications to the default energy cutoffs and methods:

*Efficient Quantum Chemical Calculation of Structure Ensembles and Free Energies for Nonrigid Molecules*
S. Grimme, F. Bohle, A. Hansen, P. Pracht, S. Spicher, and M. Stahn
J. Phys. Chem. A 2021, 125, 19, 4039-4054 (https://pubs.acs.org/doi/pdf/10.1021/acs.jpca.1c00971)
All ORCA calculations for steps 2 and 3 employ the resolution-of-the-identity (RI) and chain-of-spheres-exchange (COSX) approximation with the implemented fitting basis sets (def2/J) and default integration grids.

The following sample jobs (S#) include all keywords relating to the methods described above. However, additional keywords and settings might be required in some or all cases to avoid technical problems (e.g. SCF convergence issues). As these settings depend on the individual case, they are not provided here.

*S1: Ground-State-Optimization of Eu(II) complex (Dipole Moment 3A and Ionization Potential 3C)*

```
!wB97X-D3 def2-SVP gCP(DFT/SVP) def2/J RIJCOSX LOOSEOPT
!CPCM
%cpcm       epsilon         3
            refrac          2.25
            SMD             true
            SMDsolvent      "ANISOLE"
end
%basis newgto Eu "def2-TZVP" end
end
*xyzfile 0 8 input.xyz
```

*S2: Excited-State-Optimization of Eu(II) complex (Emission energy 3B and Ionization Potential 3D)*

```
!wB97X-D3 def2-SVP gCP(DFT/SVP) def2/J RIJCOSX LOOSEOPT
!CPCM
%cpcm       epsilon        3
            refrac         2.25
            SMD            true
            SMDsolvent     "ANISOLE"
end
%basis newgto Eu "def2-TZVP" end
end
%tddft      nroots      4
            iroot       1
            maxdim      80
            NGuessMat   2048
            maxiter     250
end
*xyzfile 0 8 input.xyz
```

*S3: Ground-State-Optimization of Eu(III) complex (Ionization Potentials 3C and 3D)*

```
!wB97X-D3 def2-SVP gCP(DFT/SVP) def2/J RIJCOSX LOOSEOPT
!CPCM
%cpcm       epsilon        3
            refrac         2.25
            SMD            true
            SMDsolvent     "ANISOLE"
end
%basis newgto Eu "def2-TZVP" end
end
*xyzfile 1 7 input.xyz
```

***I. Experiment results using the Quantum-mechanical methods described above***

*Table 1. Summary of experimental data obtained using quantum mechanical calculations.*

| # | Cation | Ligand | Anion | Complex | Molecular Dipole | Emission Energy | IP/Eu(I I) | IP/Eu(II) * |
|---|---|---|---|---|---|---|---|---|
| | ***Comparable examples from literature (Prior Art)*** | | | | | | | |
| 1 | Eu(II) | | a3 | CA1 | 32.9 | 2.84 | -5.81 | -2.68 |
| 2 | Eu(II) | | a26, with $R_b$=H | CA2 | 47.3 | 2.81 | -6.25 | -3.07 |
| 3 | Eu(II) | | a3 | CA3 | 23.3 | 2.34 | -4.81 | -1.95 |
| | *Examples according to invention* | | | | | | | |
| 6 | Eu(II) | L1 | a3 | E1 | 9.2 | 2.78 | -5.13 | -2.09 |
| 7 | Eu(II) | L2 | a3 | E9 | 11.8 | 2.75 | -5.20 | -2.20 |
| 8 | Eu(II) | L28 | a3 | E10 | 8.5 | 2.82 | -5.33 | -2.27 |
| 9 | Eu(II) | L16 | a3 | E11 | 8.7 | 2.66 | -5.04 | -2.12 |
| 10 | Eu(II) | L7 | a3 | E13 | 7.5 | 2.81 | -5.23 | -2.19 |
| 11 | Eu(II) | L6 | a3 | E12 | 4.4 | 2.88 | -5.38 | -2.28 |
| 12 | Eu(II) | L1 | a26, with $R_b$=H | E2 | 13.4 | 2.54 | -5.66 | -2.71 |
| 13 | Eu(II) | L1 | a26, with $R_b$=H, Me | E4 | 13.1 | 2.57 | -5.67 | -2.71 |
| 14 | Eu(II) | L1 | a26, with $R_b$=Me | E3 | 17.6 | 2.74 | -6.03 | -2.82 |

(continued)

| | | | | | | | | |
|---|---|---|---|---|---|---|---|---|
| | *Examples according to invention* | | | | | | | |
| 15 | Eu(II) | L1 | a41, with $R_b$=H | E5 | 12.7 | 2.70 | -5.60 | -2.59 |
| 16 | Eu(II) | L1 | a41, with $R_b$=Me | E6 | 20.3 | 2.70 | -5.99 | -2.90 |
| 17 | Eu(II) | L1 | a42, with $R_b$=H | E7 | 12.6 | 2.68 | -5.83 | -2.74 |
| 18 | Eu(II) | L1 | a43, with $R_b$=H | E8 | 38.8 | 2.67 | -5.30 | -2.07 |
| 19 | Eu(II) | L17, H | | E14 | 12.6 | 2.57 | -5.97 | -3.05 |
| 20 | Eu(II) | L18, H | | E15 | 16.7 | 2.52 | -6.09 | -2.94 |
| 21 | Eu(II) | L19, H | | E16 | 14.5 | 2.62 | -6.08 | -3.20 |
| 22 | Ce(III) | L20 | a3 | E17 | 19.6 | 3.06 | -4.88 | -1.25 |

With the above-described quantum mechanical methods, 17 metal organic coordination compounds according to the invention have been investigated in detail. The results of the examples according to invention are summarized in **Table 1** above as number 6-22. Additionally, three comparative examples, CA1-CA3, have been calculated using identical methods, depicted as number 1-3.

In this table, the second row after the numbering quotes the lanthanide cation present in the metal organic compound according to the invention. In most examples divalent Europium is used, yet trivalent Cerium is employed in example #22. The next row states the polycyclic ligand used to construct the complex. The L numbers refer to the chemical structures shown in Fig. 1. The next row denotes the anions that are part of the complex, whereby the "a" codes refer to the chemical structures depicted in Fig. 4. Finally, in the row labelled "complex" an "E" code is given, which refers to the full complexes depicted in Fig. 5. The comparative examples CA1-CA3 refer to the symmetrical aza-cryptands:

CA1

CA3

CA2

Calculations have been conducted for the lowest conformer of each complex in an environment similar to organic thin-films. The molecular dipole moment is given in Debye (D), (vertical) emission energy and ionization potentials are given in electron volt (eV). IP/Eu(II) corresponds to the depth of the potential of the most weakly bound electron in the electronic ground state of the complex, while IP/Eu(II)* refers to the potential depth of the most weakly bound electron in the lowest electronically excited state of the complex.

As apparent from the table, all emission energies of the examples according to the invention fall in the deep blue spectral region as desired for display applications. Consistent with experimental observation (compare Li et.al., Nature communications,

(2020) 11:5218), the comparative example CA3 emits in the green spectral not suited for display applications. Here, the relative flexible ligand of CA3 is not well suited to complex divalent Europium.

A further advantage of the examples according to invention is their particularly low dipole moment, which makes the complexes soluble in less polar solvents and improves their volatility, allowing for thermal vacuum coating. Here all the materials according to the invention, except #18, show dipole moments of less than 21D, while the comparative examples show higher than 21D dipole moments. The difference is particular pronounced if one only compares with the for display

application relevant deep blue emitting comparative examples CA1 and CA2, which both have dipole moments of more than 30D. The preferrable low dipole observed for the coordination compounds according to invention is an intrinsic feature of the asymmetrical nature of the polycyclic ligands according to formula (1-1) and (1-2), which allows for perfect accommodation of two anions, thereby partly canceling the high dipole moment associated with the presence of two anions and one dication. Indeed, such a desired cancellation is not possible using the symmetrical, three-armed aza cryptands employed in the comparative examples CA1-CA3. Finally, example according to the invention #18, i.e., E8 in Fig.5, does as well show a very high dipole. Here, the reason is obviously the use of the twofold negatively charged anion, a43, compare Fig. 4. Using such single anion no canceling of dipole moments is possible, dianions are thus less preferred over the use of two singly charged anions.

The energy levels are depicted in the last two rows of Table 1. Most important in this context is the IP, referring to the ionization potential of the coordination compounds. In all cases, ionization corresponds to the oxidation of the central Europium cation from the divalent to the trivalent state. Accordingly, a low IP is desired as this is an indication of good air stability, and as such allows for easy processing. This parameter is particularly important for coordination compounds using divalent Europium as central metal as here the irreversible oxidation to trivalent Europium must be prevented. All coordination compounds according to invention based on divalent Europium do show a desirable low ionization potential, IP, of <-5.0 eV, which is evidence for a very good chemical stabilization of the central divalent Europium against undesired oxidation. A surprising observation in this context is the relatively even lower IP of those coordination compounds comprising closoborane and related anions, i.e., #12-#21. For example, by exchanging just the two iodides of example #6 into various closocarborane or borane cluster anions in examples #12-17, the IP is substantially lowered from -5.13 eV to between -5.66 to -6,03 eV. Similarly, the covalent attachment of closocarborane anions on the cage used in the example #6, as performed in examples #19-21, again significantly reduces the IP to around -6eV. Thus, all those boran cluster anions significantly improve the stability of the complex towards ambient conditions and as such are preferred anions.

## II. Examples synthesized

*Synthesis of starting materials*

Europium (II) carbadodecaborate (Eu(CB)2)

Trimethylammonium carbadodecaborate (Katchem, 1 eq, 813 mg, 4 mmol) was dried in a Glovebox for 1 h at 100 °C. It was then dissolved in anhydrous THF (20 ml) and treated dropwise with a solution of bis[bis(trimethylsilyl)amido] bis(tetrahydrofuran)europium (0.5 eq, 1234 mg, 2 mmol) in anhydrous THF (10 ml) was at RT. The mixture was stirred for 1 h. White crystals formed which were collected via suction filtration, washed with THF, and dried in vacuum to yield the title compound.

p-[2-(2-{2-[2-(Tosyloxy)ethoxy]ethoxy}ethoxy)ethoxysulfonyl]toluene

The title compound was prepared using a known procedure (R.-P. Moldovan et al., J. Med. Chem. 2016, 59, p. 7840 - 7855). The product was isolated as a colorless oil and dried in vacuum before use.
Yield: 97%

4,7,10,16,21-Pentaoxa-1,13-diazabicyclo[11.5.5]tricosane **L1**

A mixture of 1,7-Diaza-12-crown-4 ether (1 eq., 5,74 mmol), p-[2-(2-{2-[2-(Tosyloxy)ethoxy]ethoxy}ethoxy) ethoxy-sulfonyl]toluene (1 eq., 5,74 mmol) and Sodium carbonate (242 eq., 1388 mmol) in MeCN (573 ml) was refluxed for 24 h. After this time, the reaction mixture was cooled to room temperature and the solvent removed under reduced pressure. The solid residue was dissolved in 920 ml water. The aqueous layer was extracted with dichloromethane. The combined organic phases were dried over magnesium sulfate. The solvent was removed under reduced pressure. The residue was filtered through a pad of aluminium oxide (neutral) with THF/EtOH (100/1, 500 ml) as eluant. The solvents were removed under reduced pressure. The crude product was purified by chromatography on aluminium oxide (neutral) using dichloromethane/methanol (0% to 3% to 5%) as eluate, yielding after concentration a slightly yellow oil.

In order to remove any sodium salts present, this oil was dissolved in methanol and acidified with hydrochloric acid (1 M). The solvents were removed under reduced pressure and the remaining white solid was passed through an anion-exchange column (Amberlite IRA-410 (Cl), 160 ml, pre-treated with 10% sodium hydroxide solution, eluant methanol/water 20/1). The collected aqueous solution was concentrated under reduced pressure and the residue extracted with hexane. The crude product was further purified by bulb-to-bulb distillation to yield the title compound as a colorless oil.
Yield: 54%
1H-NMR (61.75 MHz, CDCl3) □ (ppm) = 3.77 - 3.38 (m, 20H, CH2O), 2.65 (qd, J = 3.4, 1.8 Hz, 12H, CH2N); 13C-NMR

(15.53 MHz, CDCl3) □ (ppm) = 70.68 (OCH2CH2O), 70.52 (CH2OCH2), 70.10 (NCH2CH2O), 69.89 (NCH2CH2O), 57.26 (NCH2CH2O), 56.34 (NCH2CH2O); MS (ESI): m/z = 333 [M+H]+, 355 [M+Na]+, 371 [M+K]+.

**General procedures for preparation of metal organic compounds:**

The metal organic compounds were prepared using the known procedure described in WO2021/244801 and WO2022/218562, or using the following pathway:
The europium precursor (1 eq) and Ligand (1 eq) were dissolved separately in an appropriate solvent (THF or toluol). The solution, containing Eu(II), was added dropwise to the stirred solution of the ligand at RT. If THF only is used as the reaction solvent, the precipitated product is separated by filtration, washed with THF, and dried. In other cases, reaction mixture is evaporated to dryness, the residue triturated with diethyl ether, the precipitated product separated by filtration, washed with diethyl ether and dried.

Following this procedure, using the ligand L1 (shown in Fig. 1), $EuI_2$ and $Eu(CB)_2$ respectively, the compounds E1 and E2 (shown in **Fig 5**) were obtained in high purity.

In a similar way and closely following the procedures described in WO2021/244801 and WO2022/218562 and Chem. Commun. 2018, 54, pages 4545-4548, CA1, CA2 and CA3 have been synthesized, respectively.

The coordination compounds were further purified using a three-temperature zone sublimation tool (CreaPhys, DSU-5) being installed in a nitrogen glovebox. Here, the weight of the sublimed material was measured relative to the weight of the material in the sublimation source, giving the sublimation yield.

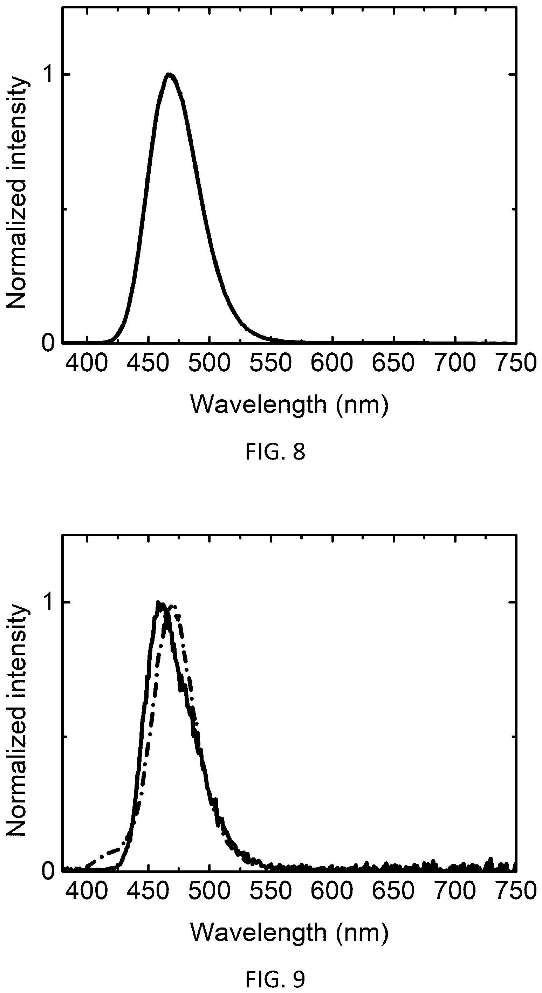

The emission spectra of E1 and E2 dissolved in water and oxygen free methanol are depicted in **Fig. 8** as solid and dashed line, respectively. Indeed, the two spectra are almost perfectly on top of each other, which is expected given the fact that the same ligand L1 is used as part of the coordination compound and the anions are supposedly solvated.

The emission spectra of E1 and E2 in solid state (as powder) are plotted in **Fig. 9** as solid and dashed line, respectively. Whilst both spectra are narrower compared to their solution counterparts, here, E2 appears red shifted in peak wavelength, relative to the E1. Still both spectra exhibit a very narrow emission peak in the deep blue spectral region ideally suited for display applications.

*Table 2 Overview of experimental data obtained with coordination compounds according to the invention vs comparative examples.*

| Compound | Emission wavelength /nm | Solubility in THF | Sublimation yield /% |
|---|---|---|---|
| CA1 | 459 | No | 0 |
| CA2 | 461 | No | 0 |
| CA3 | 518 | No | 11.4 |
| E1 | 460 | Yes | 16.3 |
| E2 | 469 | Partly | 2.6 |

**Table 2** summarizes experimental data collected for various coordination compounds according to the invention as well as for some comparative examples. Here, the second row shows the emission peak wavelength of the complexes as observed in solid state. Evidently, both E1 and E2 emit in the deep blue spectral region as desired for display applications. Indeed, the trends and to a good approximation the absolute energies are very well reproduced using the quantum mechanical methods and presented in Table 1 above. For example, CA3 is observed to emit at 518 nm in solid state, being very consistent with the value predicted by DFT methods. Thus, CA3 is not useful for display applications requiring deep blue light emission as it does not substantially emit at wavelength shorter than 500 nm.

Solubility in low polar solvents, for example quantified using the polarity index, is a desired feature of coordination compounds as described in detail above. Here, all complexes shown in Table 2 dissolve in high polar acetonitrile or methanol, both having a polarity index of 5.1, whilst only the examples according to invention as well dissolve in low polar tetrahydrofuran (THF) having a polarity index of only 4.0. Finally, high volatility is a desirable property of coordination compounds, as it allows processing using thermal evaporation or sublimation techniques. Here, the sublimation yield is given in the last column, following the definitions given above. Whilst CA3 shows some sublimation yield, it is not deep blue in emission color and as such not useful for OLED applications. On the other hand, the two deep blue comparative examples CA1 and CA2 do not sublime at all, they cannot be processed using sublimation or evaporation techniques. Yet, the two examples according to the invention, E1 and E2 based on formula (1-1), combine a reasonable sublimation yield with a deep blue emission color and as such are perfectly suited for OLED display applications, whereby the OLED devices are processed by sublimation or evaporation.

## Device examples

Representative embodiment of an organic electronic device according to various embodiments will now be described, including a detailed description of the fabrication process of the organic electronic device. Yet, it may be understood that neither the specific techniques for fabrication of the device, nor the specific device layout, nor the specific compounds are intended to limit the scope.

*Material definitions:*

ITO: Indium tin oxide
MoO3: Molybdenum trioxide
NPB: N,N'-Di(1-naphthyl)-N,N'-diphenyl-(1,1'-biphenyl)-4,4'-diamine
TAPC: 1,1-Bis[(di-4-tolylamino)phenyl]cyclohexane
TSPO1: Diphenyl[4-(triphenylsilyl)phenyl]phosphine oxide
Bphen: Bathophenanthroline
Cs: Cesium
Al: Aluminium NPB, TAPC, TSPO1, Bphen were purchased from Lumtec and used as-received.

*Device preparation:*

TAPC

Devices are exemplified with the deep blue emitting material according to formula (1-1), **E1** shown in Fig.5. TAPC was chosen as host for **E1** emitter according to the invention as it has a triplet energy of 443 nm i.e. shorter than the emission energy of **E1** being 460 nm, ensuring energy confinement on the emitter. Additionally, TAPC has a low hole and electron affinity of about -2 eV ensuring hole trapping and subsequent electron confinement on the emitter.

The organic electronic device was prepared with the following layer sequence: ITO / MoO3 2 nm / NPB 50 nm / TAPC 10 nm / TAPC:**E1** 10 wt% 20 nm / TSPO1 10 nm / BPhen 30 nm / Cs 1 nm / Al 100 nm.

The organic electronic device was fabricated on a 1"x1" size glass substrate, precoated with a transparent ITO anode. The substrate was cleaned with various solvents in an ultrasonic bath and subsequently exposed to oxygen plasma for 10 min. Following the cleaning procedure, the substrate was loaded into an ultra-high vacuum evaporation tool operating at around $10^{-7}$ mbar pressure. Here, the organic layers were deposited onto the substrate in the sequence stated above. Given the excellent sublimation yield of **E1**, compare table. 2, the generally preferred method of thermal evaporation was used to fabricate the OLED device. In the case of TAPC:**E1**, a co-evaporation process was used with both materials evaporating at the same time at different rates, corresponding to the resulting state doping ratio. The device was finished by evaporating a 100 nm film of cathode Al. The Al deposition was carried out using a structured shadow mask, such that the resulting overlap of pre-structured ITO, organic layers and Al gave an active area of 6.25 $mm^2$. Subsequently, the device was transferred to a nitrogen atmosphere glovebox and encapsulated by gluing an additional encapsulating glass substrate on top to prevent oxygen- and water-induced degradation. The electrical and electroluminescent properties of the device were measured using a Keithley 2400 source meter, OceanOptics USB4000 spectrometer and a calibrated Si photodiode.

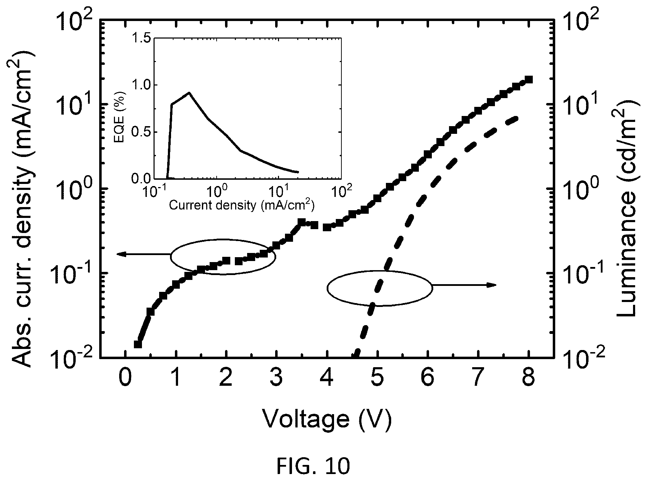

The device showed turn-on at 4.5 V, the current density reached 1 $mA/cm^2$ at 7.2 V. The OLED emitted in the deep blue spectral region with an emission spectrum almost exactly resembling the optically excited solid-state emitter shown in Fig 9, especially the peak emission wavelength remained at 460 nm. Some detailed device characteristics are presented in **FIG 10.** The current-voltage (*j-V*) curve (solid line) and the current-luminance (*j-L*) curve (dashed line) of the resulting organic electronic device are plotted in the main part of the graph. The efficiency (EQE) plotted over current density is displayed in the inset.

## Claims

1. A metal-organic coordination compound, wherein the coordination compound comprises one lanthanide ion selected from Eu(II) coordinated by a polycyclic organic ligand having the formula (1-1) or (1-2)

(1-1) (1-2)

with
$L_0$, $L_1$, $L_2$ being independently each a divalent organic group formed by removing two hydrogen atoms from an organic molecule containing at least two hydrogen atoms, with the shortest sequence of atoms in L1, and L2 each linking the two nitrogen atoms adjacent to L1, and L2 being each 5 atoms long, which are carbon atoms, with one non-terminal carbon atom of these being replaced by O, and with **R** independently in each occurrence representing hydrogen, deuterium, halogen, or a monovalent organic group formed by removing a hydrogen atom from an organic molecule containing at least one hydrogen atom, and herein any two beta- or gamma-positioned R can be linked to each other to form a ring structure.

2. The coordination compound according to claim 1, wherein $L_0$, $L_1$, and $L_2$ only contain atoms, selected from hydrogen, deuterium, carbon, nitrogen, and oxygen.

3. The coordination compound according to claim 1 or 2, wherein at least two atoms present in the shortest sequence of atoms of any of $L_0$, $L_1$, or $L_2$ independently are part of a benzene, pyridine, pyrrole, pyrazole, furan, carbazole, dibenzofurane, benzimidazole, or benzofuran ring structure, preferably furan or pyrrole, ring structure, most preferably furan ring structure.

4. The coordination compound according to one of claims 1 to 3, wherein the shortest sequences of $L_1$, and $L_2$ between the two nitrogen atoms adjacent to $L_1$ and $L_2$ are ethyleneoxy, azaethylene or dimethylfuran fragments and the polycyclic organic ligand is selected from one of formulas (1-3), (1-5), (1-6) and (1-8)

1-3

1-5

1-6

1-8

5. The coordination compound according to any of claims 1 to 4, wherein the polycyclic organic ligand is selected from

one of formulas (1-9) to (1-21)

R
O
N
1-9
1-10
1-11
1-12
1-13
1-14
1-15
1-16
1-17
1-18
1-19
1-20
1-21
BR$_b$
C
n

with

$R_b$ independently in each occurrence representing hydrogen, deuterium, halogene - preferably chlorine -, or straight or branched alkyl chain radicals including cycloalkyl radicals, the alkyl groups containing from one to fifteen, preferably one to ten, more preferably one to five carbon atoms, the term "cycloalkyl radical" referring to monocyclic, polycyclic, and spiro alkyl radicals containing 3 to 12, preferably 3 to 8 ring carbon atoms, and **n** independently in each occurrence being an integer in the range of 0 to 10, preferably 1 to 3.

**6.** The coordination compound according to any of claims 1 to 5, wherein the coordination compound comprises at least one negatively charged anion that is not covalently linked to the polycyclic organic ligand, and/or wherein the

polycyclic organic ligand, excluding the lanthanide ion, is at least singly, preferably twofold, negatively charged.

**7.** The coordination compound according to claim 6, wherein the at least one negatively charged anion is a halogene, preferably iodide or a closocarborane according to one of formulas (1-22) - (1-24):

(1-22)
(1-23)
(1-24)

with $\mathbf{R_b}$ independently in each occurrence representing hydrogen, deuterium, halogene - preferably chlorine, straight or branched chain or cyclo-$C_{1-15}$-alkyl - preferably methyl, and/or wherein the ligand contains at least one, preferably two, covalently attached closocarborane fragments according to formula (1-25) or (1-26):

(1-25)
(1-26)

wherein
the dashed line indicates the covalent linkage to the remainder of the organic ligand, and
$\mathbf{R_b}$ independently in each occurrence represents either hydrogen, deuterium, halogene - preferably chlorine -, or straight or branched alkyl chain radicals including cycloalkyl radicals, the alkyl groups containing from one to fifteen, preferably one to ten, more preferably one to five carbon atoms, the term "cycloalkyl radical" referring to monocyclic, polycyclic, and spiro alkyl radicals containing 3 to 12, preferably 3 to 8 ring carbon atoms, - preferably $C_{1-3}$-alkyl, more preferably methyl.

**8.** A mixture comprising

(a) the coordination compound according to any one of claims 1 to 7, and
(b) at least one second electrically neutral organic compound different therefrom, wherein the coordination compound (a) is imbedded into the at least one second electrically neutral organic compound (b), which preferably is a charge-neutral host material,

wherein the second organic compound (b) has a triplet energy higher than 2.5 eV, preferably higher than 2.6 eV and more preferably higher than 2.7 eV, wherein compounds (a) and (b) are physically mixed or wherein the second charge-neutral compound (b) has a molecular weight $M_n$ above 1000 g/mol and the coordination compound (a) and the second charge-neutral compound (b) are chemically linked with each other, for example by a covalent or coordinative chemical bond.

**9.** The mixture according to claim 8,

wherein the coordination compound is electrically neutral or singly positively charged and is imbedded into the at least one second electrically neutral organic compound in a ratio of 0.1 to 99 vol%, based on the mixture which is 100 vol%,
wherein the second organic compound has a lower hole affinity compared to the electrically neutral coordination compound and/or
wherein the second organic compound has a lower electron affinity compared to the singly positively charged coordination compound.

**10.** An organic electronic device, comprising:

a first electrode;
a second electrode; and
an organic layer arranged such that it is electrically interposed between the first and second electrodes, wherein the organic layer comprises the coordination compound according to one of claims 1 to 7, or the mixtures according to claim 8 or 9,
wherein the organic electronic device is preferably an organic light emitting diode, whereby preferably a substantial portion of the light is emitted at wavelengths lower than 500 nm.

**11.** The organic electronic device according to claim 10, comprising a second substantially organic layer which contains an organic material and which is in physical contact with the first organic layer, with the organic material in the second substantially organic layer

(i) having a higher electron binding energy than the charge-neutral coordination compound (a),
(ii) and/or having a higher triplet energy compared to the emission energy of the coordination compound (a) in its lowest electronically excited state,
(iii) and/or having an electron affinity that is lower than the electron binding energy of the coordination compound (a) in its lowest electronically excited state,

wherein preferably a sequence of the first organic layer and the second substantially organic layer is arranged such that they are electrically interposed between the first and second electrodes.

**12.** A method of forming an organic device according to claim 10 or 11, the method comprising:

forming a layer of the coordination compound according to any one of claims 1 to 7, or of the mixtures according to claim 8 or 9,
wherein the layer is deposited from a gas phase, preferably using an evaporation and/or sublimation and/or carrier gas process, or wherein the layer is deposited from solution.

**13.** A method of forming an organic device according to claim 12, whereby the solution is comprised substantially of organic solvents with a polarity index of less than 5.0, preferably of less than 4.0, more preferably of less than 3.0.

**14.** The use of a coordination compound according to one of claims 1 to 7 or of a mixture according to any of claims 8 or 9 as active emitting material in electroluminescent electronics applications or as a dye or contrast enhancement medium for magnetic resonance tomography.

**Patentansprüche**

**1.** Metallorganische Koordinationsverbindung, wobei die Koordinationsverbindung ein Lanthanidion umfasst, das aus Eu(II) ausgewählt ist, das durch einen polycyclischen organischen Liganden mit der Formel (1-1) oder (1-2) koordiniert ist:

(1-1) (1-2)

wobei

$L_0$, $L_1$, $L_2$ unabhängig voneinander jeweils eine zweiwertige organische Gruppe, die durch Entfernen von zwei Wasserstoffatomen von einem organischen Molekül, das mindestens zwei Wasserstoffatome enthält, gebildet wird, sind, wobei die kürzeste Abfolge von Atomen in L1 und L2, die jeweils die zwei Stickstoffatome, die L1 und L2 benachbart sind, verknüpft, jeweils 5 Atome lang ist, bei denen es sich um Kohlenstoffatome handelt, wobei ein nichtterminales Kohlenstoffatom von diesen durch O ersetzt ist, und wobei

R unabhängig bei jedem Auftreten für Wasserstoff, Deuterium, Halogen oder eine einwertige organische Gruppe, die durch Entfernen eines Wasserstoffatoms von einem organischen Molekül, das mindestens ein Wasserstoffatom enthält, gebildet wird, steht, wobei beliebige zwei R in beta- oder gamma-Position miteinander zu einer Ringstruktur verknüpft sein können.

**2.** Koordinationsverbindung nach Anspruch 1, wobei $L_0$, $L_1$ und $L_2$ nur Atome enthalten, die aus Wasserstoff, Deuterium, Kohlenstoff, Stickstoff und Sauerstoff ausgewählt sind.

**3.** Koordinationsverbindung nach Anspruch 1 oder 2, wobei mindestens zwei Atome, die in der kürzesten Abfolge von Atomen eines beliebigen von $L_0$, $L_1$ oder $L_2$ vorliegen, unabhängig Teil einer Benzol-, Pyridin-, Pyrrol-, Pyrazol-, Furan-, Carbazol-, Dibenzofuran-, Benzimidazol- oder Benzofuranringstruktur, vorzugsweise Furan- oder Pyrrolringstruktur, ganz besonders bevorzugt Furanringstruktur, sind.

**4.** Koordinationsverbindung nach einem der Ansprüche 1 bis 3, wobei die kürzesten Abfolgen von $L_1$ und $L_2$ zwischen den zwei Stickstoffatomen, die $L_1$ und $L_2$ benachbart sind, Ethylenoxy-, Azaethylen- oder Dimethylfuranfragmente sind und der polycyclische organische Ligand aus einer der Formeln (1-3), (1-5), (1-6) und (1-8) ausgewählt ist

1-3

L0
R
O
N

1-5

L0
R
O
N

1-6

L0
R
O
N

1-8

**5.** Koordinationsverbindung nach einem der Ansprüche 1 bis 4, wobei der polycyclische organische Ligand ausgewählt ist aus einer der Formeln (1-9) bis (1-21)

1-9
1-10
1-11
1-12
1-13
1-14
1-15
1-16
1-17
1-18
1-19
1-20
1-21

wobei

$R_b$ unabhängig bei jedem Auftreten für Wasserstoff, Deuterium, Halogen, vorzugsweise Chlor, oder geradkettige oder verzweigte Alkylkettenreste einschließlich Cycloalkylresten steht, wobei die Alkylgruppen eins bis fünfzehn, vorzugsweise eins bis zehn, besonders bevorzugt eins bis fünf Kohlenstoffatome enthalten, wobei sich der Begriff "Cycloalkylrest" auf monocyclische, polycyclische und Spiro-Alkylreste mit 3 bis 12, vorzugsweise 3 bis 8, Ringkohlenstoffatomen bezieht, und

n unabhängig bei jedem Auftreten eine ganze Zahl im Bereich von 0 bis 10, vorzugsweise 1 bis 3, ist.

6. Koordinationsverbindung nach einem der Ansprüche 1 bis 5, wobei die Koordinationsverbindung mindestens ein negativ geladenes Anion umfasst, das nicht kovalent mit dem polycyclischen organischen Liganden verknüpft ist, und/oder wobei der polycyclische organische Ligand unter Ausschluss des Lanthanidions mindestens einfach, vorzugsweise zweifach, negativ geladen ist.

7. Koordinationsverbindung nach Anspruch 6, wobei das mindestens eine negativ geladene Anion ein Halogen,

vorzugsweise Iodid, oder ein Closocarboran gemäß einer der Formeln (1-22) - (1-24) ist:

(1-22)
(1-23)
(1-24)

wobei $R_b$ unabhängig bei jedem Auftreten für Wasserstoff, Deuterium, Halogen, vorzugsweise Chlor, gerad- oder verzweigtkettiges oder Cyclo-$C_{1-15}$-alkyl, vorzugsweise Methyl, steht, und/oder wobei der Ligand mindestens ein, vorzugsweise zwei, kovalent gebundene Closocarboranfragmente gemäß Formel (1-25) oder (1-26) enthält:

(1-25)
(1-26)

wobei
die gestrichelte Linie die kovalente Verknüpfung mit dem Rest des organischen Liganden anzeigt und
$R_b$ unabhängig bei jedem Auftreten entweder für Wasserstoff, Deuterium, Halogen, vorzugsweise Chlor, oder geradkettige oder verzweigte Alkylkettenreste einschließlich Cycloalkylresten steht, wobei die Alkylgruppen eins bis fünfzehn, vorzugsweise eins bis zehn, besonders bevorzugt eins bis fünf Kohlenstoffatome enthalten, wobei sich der Begriff "Cycloalkylrest" auf monocyclische, polycyclische und Spiro-Alkylreste mit 3 bis 12, vorzugsweise 3 bis 8, Ringkohlenstoffatomen bezieht, vorzugsweise $C_{1-3}$-Alkyl, weiter bevorzugt Methyl.

**8.** Mischung, umfassend

(a) die Koordinationsverbindung nach einem der Ansprüche 1 bis 7 und
(b) mindestens eine davon verschiedene zweite elektrisch neutrale organische Verbindung,
wobei die Koordinationsverbindung (a) in die mindestens eine zweite elektrisch neutrale organische Verbindung (b), bei der es sich vorzugsweise um ein ladungsneutrales Wirtsmaterial handelt, eingebettet ist,
wobei die zweite ladungsneutrale organische Verbindung (b) eine Triplettenergie von mehr als 2,5 eV, vorzugsweise mehr als 2,6 eV und weiter bevorzugt mehr als 2,7 eV aufweist, wobei die Verbindungen (a) und (b) physikalisch gemischt sind oder wobei die zweite ladungsneutrale Verbindung (b) ein Molekulargewicht $M_n$ über 1000 g/mol aufweist und die Koordinationsverbindung (a) und die zweite ladungsneutrale Verbindung (b) chemisch miteinander verknüpft sind, zum Beispiel über eine kovalente oder koordinative chemische Bindung.

**9.** Mischung nach Anspruch 8,

wobei die Koordinationsverbindung (a) elektrisch neutral oder einfach positiv geladen ist und in einem Anteil von 0,1 bis 99 Vol.-%, bezogen auf die Mischung, die 100 Vol.-% beträgt, in die mindestens eine zweite elektrisch neutrale organische Verbindung eingebettet ist,

wobei die zweite organische Verbindung eine geringere Lochaffinität als die elektrisch neutrale Koordinationsverbindung aufweist und/oder wobei die zweite organische Verbindung eine geringere Elektronenaffinität als die einfach positiv geladene Koordinationsverbindung aufweist.

**10.** Organische elektronische Vorrichtung, umfassend:

eine erste Elektrode;
eine zweite Elektrode und
eine organische Schicht, die so angeordnet ist, dass sie sich elektrisch zwischen der ersten und der zweiten Elektrode befindet, wobei die organische Schicht die Koordinationsverbindung nach einem der Ansprüche 1 bis 7 oder die Mischungen nach Anspruch 8 oder 9 umfasst,
wobei es sich bei der organischen elektronischen Vorrichtung vorzugsweise um eine organische Leuchtdiode handelt, wobei vorzugsweise ein wesentlicher Teil des Lichts bei Wellenlängen kleiner als 500 nm emittiert wird.

**11.** Organische elektronische Vorrichtung nach Anspruch 10, umfassend eine zweite weitgehend organische Schicht, die ein organisches Material enthält und die in physischem Kontakt mit der ersten organischen Schicht steht, wobei das organische Material in der zweiten weitgehend organischen Schicht ist

(i) eine höhere Elektronenbindungsenergie als die ladungsneutrale Koordinationsverbindung (a) aufweist
(ii) und/oder eine höhere Triplettenergie im Vergleich zu der Emissionsenergie der Koordinationsverbindung (a) in ihrem niedrigsten elektronisch angeregten Zustand aufweist
(iii) und/oder eine Elektronenaffinität aufweist, die niedriger als die Elektronenbindungsenergie der Koordinationsverbindung (a) in ihrem niedrigsten elektronisch angeregten Zustand ist,

wobei vorzugsweise eine Abfolge der ersten organischen Schicht und der zweiten weitgehend organischen Schicht so angeordnet ist, dass sie sich elektrisch zwischen der ersten und der zweiten Elektrode befinden.

**12.** Verfahren zur Bildung einer organischen Vorrichtung nach Anspruch 10 oder 11, wobei das Verfahren umfasst:

Bilden einer Schicht aus der Koordinationsverbindung nach einem der Ansprüche 1 bis 7 oder der Mischungen nach Anspruch 8 oder 9,
wobei die Schicht aus einer Gasphase abgeschieden wird, vorzugsweise unter Verwendung eines Verdampfungs- und/oder Sublimations- und/oder Trägergasverfahrens, oder wobei die Schicht aus Lösung abgeschieden wird.

**13.** Verfahren zur Bildung einer organischen Vorrichtung nach Anspruch 12, wobei die Lösung weitgehend aus organischen Lösungsmitteln mit einem Polaritätsindex von weniger als 5,0, vorzugsweise von weniger als 4,0, weiter bevorzugt von weniger als 3,0, besteht.

**14.** Verwendung einer Koordinationsverbindung nach einem der Ansprüche 1 bis 7 oder einer Mischung nach Anspruch 8 oder 9 als aktives emittierendes Material in Elektrolumineszenz-Elektronikanwendungen oder als Farbstoff oder Kontrastverstärkungsmedium für die Magnetresonanztomographie.

## Revendications

**1.** Composé de coordination métal-organique, dans lequel le composé de coordination comprend un ion lanthanide choisi parmi Eu(II) coordiné par un ligand organique polycyclique ayant la formule (1-1) ou (1-2)

(1-1) (1-2)

$\mathbf{L_0}$, $\mathbf{L_1}$, $\mathbf{L_2}$ étant indépendamment chacun un groupe organique divalent formé par élimination de deux atomes d'hydrogène d'une molécule organique contenant au moins deux atomes d'hydrogène, la séquence d'atomes la plus courte dans L1, et L2 reliant chacun les deux atomes d'azote adjacents à L1, et L2 étant chacun de 5 atomes de longueur, qui sont des atomes de carbone, dont un atome de carbone non terminal de ceux-ci est remplacé par O, et

**R** représentant indépendamment en chaque occurrence hydrogène, deutérium, halogène, ou un groupe organique monovalent formé par l'élimination d'un atome d'hydrogène d'une molécule organique contenant au moins un atome d'hydrogène, et dans ce cas deux quelconques R positionnés en bêta ou gamma peuvent être liés l'un à l'autre pour former une structure cyclique.

**2.** Composé de coordination selon la revendication 1, dans lequel $\mathbf{L_0}$, $\mathbf{L_1}$ et $\mathbf{L_2}$ contiennent uniquement des atomes choisis parmi hydrogène, deutérium, carbone, azote, et oxygène.

**3.** Composé de coordination selon la revendication 1 ou 2, dans lequel au moins deux atomes présents dans la séquence d'atomes la plus courte de l'un quelconque parmi $\mathbf{L_0}$, $\mathbf{L_1}$ ou $\mathbf{L_2}$ font indépendamment partie d'une structure cyclique de benzène, pyridine, pyrrole, pyrazole, furanne, carbazole, dibenzofuranne, benzimidazole ou benzofuranne, de préférence une structure cyclique de furanne ou pyrrole, le plus préférablement une structure cyclique de furanne.

**4.** Composé de coordination selon l'une des revendications 1 à 3, dans lequel les séquences les plus courtes de $\mathbf{L_1}$ et $\mathbf{L_2}$ entre les deux atomes d'azote adjacents à $\mathbf{L_1}$ et $\mathbf{L_2}$ sont des fragments éthylèneoxy, azaéthylène ou diméthylfurane et le ligand organique polycyclique est choisi parmi l'une des formules (1-3), (1-5), (1-6) et (1-8)

1-3

1-5

1-6

1-8

**5.** Composé de coordination selon l'une quelconque des revendications 1 à 4, dans lequel le ligand organique polycyclique est choisi parmi l'une des formules (1-9) à (1-21)

1-9
1-10
1-11
1-12
1-13
1-14
1-15
1-16
1-17
1-18
1-19
1-20
1-21

**$R_b$** représentant indépendamment en chaque occurrence hydrogène, deutérium, halogène, - de préférence chlore -, ou des radicaux à chaîne alkyle droite ou ramifiée incluant des radicaux cycloalkyle, les groupes alkyle contenant de un à quinze, de préférence un à dix, plus préférablement un à cinq atomes de carbone, les termes « radical cycloalkyle » désignant des radicaux alkyle monocycliques, polycycliques et spiro contenant 3 à 12, de préférence 3 à 8 atomes de carbone de cycle, et

**n** étant indépendamment en chaque occurrence un entier dans la plage de 0 à 10, de préférence 1 à 3.

**6.** Composé de coordination selon l'une quelconque des revendications 1 à 5, dans lequel le composé de coordination comprend au moins un anion chargé négativement qui n'est pas lié de manière covalente au ligand organique polycyclique, et/ou dans lequel le ligand organique polycyclique, à l'exclusion de l'ion lanthanide, est chargé négativement au moins une fois, de préférence deux fois.

**7.** Composé de coordination selon la revendication 6, dans lequel l'au moins un anion chargé négativement est un halogène, préférablement un iodure ou un closocarborane selon l'une des formules (1-22) à (1-24):

(1-22)
(1-23)
(1-24)

$\mathbf{R_b}$, indépendamment, en chaque occurrence, représentant hydrogène, deutérium, halogène - de préférence chlore, alkyle à chaîne droite ou ramifiée en $C_{1-15}$ ou cyclo-(alkyle en $C_{1-15}$) - de préférence méthyle, et/ou le ligand contenant au moins un, de préférence deux, fragments closocarborane liés de façon covalente selon la formule (1-25) ou (1-26):

(1-25)
(1-26)

dans lesquelles
la ligne en pointillé indique la liaison covalente avec le reste du ligand organique, et
$\mathbf{R_b}$ représente indépendamment en chaque occurrence hydrogène, deutérium, halogène - de préférence chlore -, ou des radicaux à chaîne alkyle droite ou ramifiée y compris des radicaux cycloalkyle, les groupes alkyle contenant un à quinze, de préférence un à dix, plus préférablement un à cinq atomes de carbone, les termes « radical cycloalkyle » faisant référence à des radicaux alkyle monocycliques, polycycliques et spiro contenant 3 à 12, de préférence 3 à 8 atomes de carbone de cycle, de préférence $C_{1-3}$-alkyle, plus préférablement méthyle.

**8.** Mélange comprenant

(a) le composé de coordination selon l'une quelconque des revendications 1 à 7, et
(b) au moins un deuxième composé organique électriquement neutre différent de celui-ci,
dans lequel le composé de coordination (a) est incorporé dans l'au moins un deuxième composé organique électriquement neutre (b), qui est préférablement un matériau hôte à charge neutre,
dans lequel le second composé (b) a une énergie triplet supérieure à 2,5 eV, de préférence supérieure à 2,6 eV et plus préférablement supérieure à 2,7 eV, dans lequel les composés (a) et (b) étant physiquement mélangés ou le second composé de charge neutre (b) ayant un poids moléculaire $M_n$ supérieur à 1 000 g/mol et le composé de coordination (a) et le deuxième composé de charge neutre (b) étant chimiquement liés l'un à l'autre, par exemple par une liaison chimique covalente ou coordinante.

**9.** Mélange selon la revendication 8,

le composé de coordination étant électriquement neutre ou chargé positivement une seule fois et étant incorporé dans l'au moins un deuxième composé organique électriquement neutre en une proportion de 0,1 à 99 % en volume, par rapport au mélange qui est de 100 % en volume; le deuxième composé organique ayant une affinité de trous plus faible que le composé de coordination électriquement neutre et/ou

le deuxième composé organique ayant une affinité pour les électrons plus faible que le composé de coordination portant une seule charge positive.

**10.** Dispositif électronique organique, comprenant :

une première électrode;
une deuxième électrode; et
une couche organique agencée de sorte qu'elle est interposée électriquement entre la première et la deuxième électrode, la couche organique comprenant le composé de coordination selon l'une des revendications 1 à 7 ou les mélanges selon la revendication 8 ou 9,
préférablement le dispositif électronique organique étant une diode émettrice de lumière organique, préférablement une partie substantielle de la lumière étant émise à des longueurs d'onde inférieures à 500 nm.

**11.** Dispositif électronique organique selon la revendication 10, comprenant une seconde couche sensiblement organique qui contient un matériau organique et qui est en contact physique avec la première couche organique, avec le matériau organique dans la seconde couche sensiblement organique

(i) ayant une énergie de liaison électronique plus élevée que celle du composé de coordination neutre (a),
(ii) et/ou ayant une énergie de triplet plus élevée par comparaison à l'énergie d'émission du composé de coordination (a) dans son état électroniquement excité le plus bas,
(iii) et/ou ayant une affinité électronique inférieure à l'énergie de liaison électronique du composé de coordination (a) dans son état d'excitation électronique le plus bas,

de préférence, une séquence de la première couche organique et de la seconde couche sensiblement organique étant agencée de telle sorte qu'elles sont électriquement interposées entre les première et seconde électrodes.

**12.** Procédé de formation d'un dispositif organique selon la revendication 10 ou 11, le procédé comprenant:

la formation d'une couche du composé de coordination selon l'une quelconque des revendications 1 à 7, ou des mélanges selon la revendication 8 ou 9,
dans lequel la couche est déposée à partir d'une phase gazeuse, préférablement en utilisant un processus d'évaporation et/ou sublimation et/ou à gaz porteur, ou dans lequel la couche est déposée à partir d'une solution.

**13.** Procédé de formation d'un dispositif organique selon la revendication 12, dans lequel la solution est constituée sensiblement de solvants organiques ayant un indice de polarité inférieur à 5,0, de préférence inférieur à 4,0, plus préférablement inférieur à 3,0.

**14.** Utilisation d'un composé de coordination selon l'une des revendications 1 à 7 ou d'un mélange selon l'une quelconque des revendications 8 ou 9 en tant que matériau émetteur actif dans des applications électroniques électroluminescentes ou en tant que colorant ou agent améliorant le contraste pour la tomographie par résonance magnétique.

L 1
L 2
L 3
L 4
L 5
L 6
L 7
L 8
L 9
L 10
L 11
L 12
L 13
L 14
L 15
L 16
L 17
● = BH or BMe

FIG. 1

O
O
O
N
O
N
O
● = BH or BMe
L 18
L 19
N
H
L 20
L 21
L 22
L 23
L 24
L 25
L 26
L 27
L 28
L 29

FIG. 1 continued

L 30
L 31
L 32
L 33
L 34
L 35
L 36
L 37
L 38
L 39
L 40
L 41
L 42
L 43
L 44
L 45
L 46
L 47

FIG. 1 continued

L 48
L 49
L 50
L 51
L 52
L 53
L 54
L 55
L 56
L 57
L 58
L 59
L 60
L 61
L 62
L 63
L 64
L 65

FIG. 1 continued

L 66

L 67

L 68

L 69

L 70

L 71

L 72

L 73

L 74

L 75

L 76

L 77

L 78

L 79

L 80

FIG. 1 continued

L 81
L 82
L 83
L 84
L 85
L 86
L 87
L 88
L 89
L 90
L 91
L 92
L 93
L 94
L 95
L 96
L 97
L 98
L 99
L 100


FIG. 1 continued

HO
$F_3C$
$F_3C$
HO
$CF_3$
$CF_3$
L 101
L 102
L 103
L 104
L 105
L 106

FIG. 1 continued

- s1: --H
- s2: --D
- s3: --Cl
- s4: --F
- s5: --Br
- s6: --CN
- s7: $--NO_2$
- s8: $--CH_3$
- s9: $--CD_3$
- s10: $--CF_3$
- s11: $--CH_2\text{-}CH_3$
- s12: $--CF_2\text{-}CF_3$
- s13: $--CF(CF_3)\text{-}CF_3$
- s14: $--CH(CF_3)\text{-}CF_3$
- s15: $--CD(CF_3)\text{-}CF_3$
- s16: $--CH(CH_3)\text{-}CH_3$
- s17: $--CD(CH_3)\text{-}CH_3$
- s18: $--CD(CD_3)\text{-}CD_3$
- s19: $--C(CH_3)_2\text{-}CH_3$
- s20
- s21
- s22
- s23
- s24
- s25
- s26: $CF_3$, $CF_3$
- s27
- s28: $CF_3$, $CF_3$, $CF_3$
- s29: SiH
- s30: Si
- s31: Si
- s32: Si
- s33: Si
- s34: Si
- s35: O
- s36: O
- s37: Si, $CF_3$
- s38: $CF_3$, Si, $CF_3$
- s39: O, $CF_3$
- s40: O, $CF_3$
- s41: O, $CF_3$
- s42: Si, $CF_3$
- s43: O, C, $F_2$, $CF_3$
- s44: O, C, $F_2$, $CF_3$
- s45
- s46
- s47: N
- s48: O, S, O
- s49: O, S, O
- s50: P=O
- s51: $--SiH_2\text{-}CH_3$
- s52: $--SiH(CH_3)\text{-}CH_3$
- s53: $--Si(CH_3)_2\text{-}CH_3$
- s54: $--SO_2\text{-}CH_3$
- s55: $--SO_2\text{-}CF_3$
- s56: --P=O
- s56: --P=O, O, O
- s57: --P=O, O, O
- s58: $--CH_2D$
- s59: $--CHD_2$
- s60: O, O, $CF_3$
- s61: O, C, $F_2$, $CF_3$
- s62: O, O, C, $F_2$, $CF_3$
- s63: O
- s64: O, O
- s65: O
- s66: O, O
- s67: O
- s68: O
- s69: O
- s70: O
- s71: O

FIG. 2

b1
b2
b3
b4
b5
b6
b7
b8
b9
b10
b11
b12
b13
b14
b15
b16
b17
b18
b19
b20
b21
b22
b23
b24
b25
b26
b27
b22
b23

FIG. 3

$Cl^-$ $Br^-$ $I^-$ $ClO_4^-$ $PF_6^-$ $HSO_4^-$ $BF_4^-$ $SH^-$ $NO_3^-$ $SbF_6^-$ $AlH_4^-$ $AuCl_4^-$

**a1 a2 a3 a4 a5 a6 a7 a8 a9 a10 a11 a12**

$BH_4^-$ $C_{60}F_{48}^-$ $C_{60}F_{36}^-$

**a13 a14 a15 a16 a17 a18 a19 a20**

**a21 a22 a23**

**a24 a25 a26 a27 a28 a29**

$SO_4^{2-}$ $S_2^{2-}$ $PO_4^{3-}$ $BF_4^-$

**a30 a31 a32 a33 a34 a35 a36 a37 a38 a39**

**a40 a41 a42 a43**

FIG. 4

I⁻
Eu2+
E 1
= BH
E 2
= B
E 3
E 4
E 5
E 6
E 7
2-
E 8

FIG. 5

I⁻
Eu2+
E 9
I⁻
Eu2+
I⁻
I⁻
E 10
I⁻
Eu2+
I⁻
E 11
I⁻
Eu2+
I⁻
E 12
I⁻
Eu2+
I⁻
E 13
Eu2+
● = BH
E 14
Eu2+
● = BH
E 15
Eu2+
● = BH
E 16
I⁻
Ce3+
I⁻
E 17

FIG. 5 continued

Br⁻
Eu2+
Br⁻
Cl⁻
Eu2+
Cl⁻
BH4⁻
Eu2+
BH4⁻
E 18
E 19
E 20
E 21
E 22
E 23
E 24
E 25
E 26
E 27
E 28
E 29
E 30
F3C
CF3
N
O

FIG. 5 continued

E 31

E 32

E 33

E 34

E 35

E 36

E 37

E 38

E 39

E 40

E 41

E 42

E 43

FIG. 5 continued

I⁻
Eu2+
O
O
O
N
D
N
D
N
N
I⁻
I⁻
Ce3+
I⁻
I⁻
Ce
I⁻
Ce3+
E 44
E 45
E 46
E 47

FIG. 5 continued

100
108
106
104
102
110

FIG 6

100
106
108
104
102

FIG 7

Normalized intensity
1
0
400
450
500
550
600
650
700
750
Wavelength (nm)

FIG. 8

Normalized intensity
1
0
400
450
500
550
600
650
700
750
Wavelength (nm)

FIG. 9

Abs. curr. density (mA/cm²)
Luminance (cd/m²)
Voltage (V)
EQE (%)
Current density (mA/cm²)


FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021244801 A1
- WO 2022218562 A
- WO 2021244801 A


### Non-patent literature cited in the description


- *Chem. Commun.*, 2018, vol. 54, 4545-4548
- **R. W. TAYLOR et al.** *J. Chem. Crystallogr*, 2012, vol. 42, 573-577
- **B. VALTONCOLI et al.** *J. Chem. Soc. Perkin Trans.*, 1994, vol. 2, 815-820
- *J. Chem. Soc. Perkin Trans*, 1994, vol. 2, 3581-3588
- **J. JURCZAK et al.** *SYNTHESIS*, 2004, vol. 3, 0369-0372
- **R. W. TAYLOR et al.** *Acta Cryst.*, 2003, vol. 2008, 64
- **ROMANOVSKII et al.** Phosphorescence of π-conjugated oligomers and polymers. *Physical Review Letters*, 2000, vol. 84 (5), 1027
- **R.J.COX**. Photographic Sensitivity. Academic Press, 1973
- **S. GRIMME, F. BOHLE, A. HANSEN, P. PRACHT, S. SPICHER, AND M. STAHN**. Efficient Quantum Chemical Calculation of Structure Ensembles and Free Energies. *J. Phys. Chem. A*, 2021, vol. 125 (19), 4039-4054, https://pubs.acs.org/doi/pdf/10.1021/acs.jpca.1c00971
- **LI**. *Nature communications*, 2020, vol. 11, 5218
- **R.-P. MOLDOVAN et al.** *J. Med. Chem.*, 2016, vol. 59, 7840-7855